# EUROPEAN PATENT SPECIFICATION

(11) **EP 1 644 034 B1**
(45) Date of publication and mention of the grant of the patent: **12.01.2011**
(21) Application number: 04763176.7
(22) Date of filing: 12.07.2004
(51) Int. Cl.: A61K 39/39, A61K 39/00, A61K 39/002, A61K 39/02, A61K 39/12, A61K 39/295, A61K 39/35, A61K 39/36, A61P 37/04, C07K 14/025, C07K 14/08, C12N 15/12

(54) **COMPOSITION FOR ENHANCING AN IMMUNE RESPONSE COMPRISING PACKAGED VIRUS-LIKE PARTICLES**
ZUSAMMENSETZUNG VERPACKTE VIRUSARTIGE TEILCHEN UMFASSEND ZUR VERSTÄRKUNG EINER IMMUNANTWORT
COMPOSE DE PARTICULES DE TYPE VIRAL ENROBEES POUR AUGMENTER LA REPONSE IMMUNITAIRE

(30) Priority: 10.07.2003 US 485717 P
(43) Date of publication of application: 12.04.2006
(73) Proprietor: Cytos Biotechnology AG, 8952 Schlieren (CH)
(72) Inventor: BACHMANN, Martin, F., CH-8472 Seuzach (CH); SCHWARZ, Katrin, 8952 Schlieren (CH)
(74) Representative: Sperrle, Martin
(86) International application number: PCT/EP2004/007679
(87) International publication number: WO 2005/004907

(56) References cited:
- WO-A-01/22972
- WO-A-02/056905
- WO-A-03/024481
- SCHWARZ KATRIN ET AL: "Role of toll-like receptors in costimulating cytotoxic T cell responses." EUROPEAN JOURNAL OF IMMUNOLOGY, vol. 33, no. 6, June 2003 (2003-06), pages 1465-1470, XP008039398 ISSN: 0014-2980
- TAKEDA, K. ET AL.: "Toll-like receptors" ANNUAL REVIEW OF IMMUNOLOG, vol. 21, 1 January 2003 (2003-01-01), pages 335-376, XPXP002470283
- TAKEDA, K. ET AL.: "Toll-like receptors" ANNUAL REVIEW OF IMMUNOLOG, vol. 21, 1 January 2003 (2003-01-01), pages 335-376, XPXP002470283
- O'NEILL, L.A.J.: "How Toll-like receptors signal: what we know and what we don't know." CURRENT OPINION IN IMMUNOLOGY, vol. 18, no. 1, February 2006 (2006-02), pages 3-9, XP005237659 ENGLAND
- MEYER, T. & STOCKFLETH, E.: "Clinical investigations of Toll-like receptor agonists." EXPERT OPINION ON INVESTIGATIONAL DRUGS ENGLAND JUL 2008EXPERT OPINION ON INVESTIGATIONAL DRUGS ENGLAND JUL 2008, vol. 17, no. 7, July 2008 (2008-07), pages 1051-1065,

### SEQUENCE LISTING

<110> Cytos Biotechnology AG
   Bachmann, Martin F
   Schwarz, Katrin
<120> PACKAGED VIRUS-LIKE PARTICLES
<130> C62863PC
<150> US 60/485,717
   <151> 2003-07-10
<160> 60
<170> PatentIn version 3.2
<210> 1
   <211> 132
   <212> PRT
   <213> Bacteriophage Q-beta
<400> 1
   Ala Lys Leu Glu Thr Val Thr Leu Gly Asn Ile Gly Lys Asp Gly Lys
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65                  70                  75                  80
   Asp Pro Ser Val Thr Arg Gln Ala Tyr Ala Asp Val Thr Phe Ser Phe
                   85                  90                  95
   Thr Gln Tyr Ser Thr Asp Glu Glu Arg Ala Phe Val Arg Thr Glu Leu
               100                 105                 110
   Ala Ala Leu Leu Ala Ser Pro Leu Leu Ile Asp Ala Ile Asp Gln Leu
           115                 120                 125
   Asn Pro Ala Tyr
       130
<210> 2
   <211> 329
   <212> PRT
   <213> Bacteriophage Q-beta
<400> 2
   Met Ala Lys Leu Glu Thr Val Thr Leu Gly Asn Ile Gly Lys Asp Gly
   1 5 10 15
   Lys Gln Thr Leu Val Leu Asn Pro Arg Gly Val Asn Pro Thr Asn Gly
   20 25 30
   Val Ala Ser Leu Ser Gln Ala Gly Ala Val Pro Ala Leu Glu Lys Arg
   35 40 45
   Val Thr Val Ser Val Ser Gln Pro Ser Arg Asn Arg Lys Asn Tyr Lys
   50 55 60
   Val Gln Val Lys Ile Gln Asn Pro Thr Ala Cys Thr Ala Asn Gly Ser
   65 70 75 80
   Cys Asp Pro Ser Val Thr Arg Gln Ala Tyr Ala Asp Val Thr Phe Ser
   85 90 95
   Phe Thr Gln Tyr Ser Thr Asp Glu Glu Arg Ala Phe Val Arg Thr Glu
   100 105 110
   Leu Ala Ala Leu Leu Ala Ser Pro Leu Leu Ile Asp Ala Ile Asp Gln
   115 120 125
   Leu Asn Pro Ala Tyr Trp Thr Leu Leu Ile Ala Gly Gly Gly Ser Gly
   130 135 140
   Ser Lys Pro Asp Pro Val Ile Pro Asp Pro Pro Ile Asp Pro Pro Pro
   145 150 155 160
   Gly Thr Gly Lys Tyr Thr Cys Pro Phe Ala Ile Trp Ser Leu Glu Glu
   165 170 175
   Val Tyr Glu Pro Pro Thr Lys Asn Arg Pro Trp Pro Ile Tyr Asn Ala
   180 185 190
   Val Glu Leu Gln Pro Arg Glu Phe Asp Val Ala Leu Lys Asp Leu Leu
   195 200 205
   Gly Asn Thr Lys Trp Arg Asp Trp Asp Ser Arg Leu Ser Tyr Thr Thr
   210 215 220
   Phe Arg Gly Cys Arg Gly Asn Gly Tyr Ile Asp Leu Asp Ala Thr Tyr
   225 230 235 240
   Leu Ala Thr Asp Gln Ala Met Arg Asp Gln Lys Tyr Asp Ile Arg Glu
   245 250 255
   Gly Lys Lys Pro Gly Ala Phe Gly Asn Ile Glu Arg Phe Ile Tyr Leu
   260 265 270
   Lys Ser Ile Asn Ala Tyr Cys Ser Leu Ser Asp Ile Ala Ala Tyr His
   275 280 285
   Ala Asp Gly Val Ile Val Gly Phe Trp Arg Asp Pro Ser Ser Gly Gly
   290 295 300
   Ala Ile Pro Phe Asp Phe Thr Lys Phe Asp Lys Thr Lys Cys Pro Ile
   305 310 315 320
   Gln Ala Val Ile Val Val Pro Arg Ala
   325
<210> 3
   <211> 128
   <212> PRT
   <213> Bacteriophage PP7
<400> 3
   Met Ser Lys Thr Ile Val Leu Ser Val Gly Glu Ala Thr Arg Thr Leu
   1               5                   10                  15
   Thr Glu Ile Gln Ser Thr Ala Asp Arg Gln Ile Phe Glu Glu Lys Val
               20                  25                  30
   Gly Pro Leu Val Gly Arg Leu Arg Leu Thr Ala Ser Leu Arg Gln Asn
           35                  40                  45
   Gly Ala Lys Thr Ala Tyr Arg Val Asn Leu Lys Leu Asp Gln Ala Asp
       50                  55                  60
   Val Val Asp Cys Ser Thr Ser Val Cys Gly Glu Leu Pro Lys Val Arg
   65                  70                  75                  80
   Tyr Thr Gln Val Trp Ser His Asp Val Thr Ile Val Ala Asn Ser Thr
                   85                  90                  95
   Glu Ala Ser Arg Lys Ser Leu Tyr Asp Leu Thr Lys Ser Leu Val Ala
               100                 105                 110
   Thr Ser Gln Val Glu Asp Leu Val Val Asn Leu Val Pro Leu Gly Arg
           115                 120                 125
<210> 4
   <211> 132
   <212> PRT
   <213> Artificial Sequence
<220>
   <223> Bacteriophage Qbeta 240 mutant
<400> 4
   Ala Lys Leu Glu Thr Val Thr Leu Gly Asn Ile Gly Arg Asp Gly Lys
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 5
   <211> 132
   <212> PRT
   <213> Artificial Sequence
<220>
   <223> Bacteriophage Q-beta 243 mutant
<400> 5
   Ala Lys Leu Glu Thr Val Thr Leu Gly Lys Ile Gly Lys Asp Gly Lys
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 6
   <211> 132
   <212> PRT
   <213> Artificial Sequence
<220>
   <223> Bacteriophage Q-beta 250 mutant
<400> 6
   Ala Arg Leu Glu Thr Val Thr Leu Gly Asn Ile Gly Arg Asp Gly Lys
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 7
   <211> 132
   <212> PRT
   <213> Artificial Sequence
<220>
   <223> Bacteriophage Q-beta 251 mutant
<400> 7
   Ala Lys Leu Glu Thr Val Thr Leu Gly Asn Ile Gly Lys Asp Gly Arg
   1               5                   10                  15
   Gln Thr Leu Val Leu Asn Pro Arg Gly Val Asn Pro Thr Asn Gly Val
               20                  25                  30
   Ala Ser Leu Ser Gln Ala Gly Ala Val Pro Ala Leu Glu Lys Arg Val
           35                  40                  45
   Thr Val Ser Val Ser Gln Pro Ser Arg Asn Arg Lys Asn Tyr Lys Val
       50                  55                  60
   Gln Val Lys Ile Gln Asn Pro Thr Ala Cys Thr Ala Asn Gly Ser Cys
   65                  70                  75                  80
   Asp Pro Ser Val Thr Arg Gln Lys Tyr Ala Asp Val Thr Phe Ser Phe
                   85                  90                  95
   Thr Gln Tyr Ser Thr Asp Glu Glu Arg Ala Phe Val Arg Thr Glu Leu
               100                 105                 110
   Ala Ala Leu Leu Ala Ser Pro Leu Leu Ile Asp Ala Ile Asp Gln Leu
           115                 120                 125
   Asn Pro Ala Tyr
       130
<210> 8
   <211> 132
   <212> PRT
   <213> Artificial Sequence
<220>
   <223> Bacteriophage Q-beta 259 mutant
<400> 8
   Ala Arg Leu Glu Thr Val Thr Leu Gly Asn Ile Gly Lys Asp Gly Arg
   1 5 10 15
   Gln Thr Leu Val Leu Asn Pro Arg Gly Val Asn Pro Thr Asn Gly Val
   20 25 30
   Ala Ser Leu Ser Gln Ala Gly Ala Val Pro Ala Leu Glu Lys Arg Val
   35 40 45
   Thr Val Ser Val Ser Gln Pro Ser Arg Asn Arg Lys Asn Tyr Lys Val
   50 55 60
   Gln Val Lys Ile Gln Asn Pro Thr Ala Cys Thr Ala Asn Gly Ser Cys
   65 70 75 80
   Asp Pro Ser Val Thr Arg Gln Lys Tyr Ala Asp Val Thr Phe Ser Phe
   85 90 95
   Thr Gln Tyr Ser Thr Asp Glu Glu Arg Ala Phe Val Arg Thr Glu Leu
   100 105 110
   Ala Ala Leu Leu Ala Ser Pro Leu Leu Ile Asp Ala Ile Asp Gln Leu
   115 120 125
   Asn Pro Ala Tyr
   130
<210> 9
   <211> 185
   <212> PRT
   <213> Hepatitis B virus
<400> 9
   Met Asp Ile Asp Pro Tyr Lys Glu Phe Gly Ala Thr Val Glu Leu Leu
   1 5 10 15
   Ser Phe Leu Pro Ser Asp Phe Phe Pro Ser Val Arg Asp Leu Leu Asp
   20 25 30
   Thr Ala Ser Ala Leu Tyr Arg Glu Ala Leu Glu Ser Pro Glu His Cys
   35 40 45
   Ser Pro His His Thr Ala Leu Arg Gln Ala Ile Leu Cys Trp Gly Glu
   50 55 60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys
               180                 185
<210> 10
   <211> 185
   <212> PRT
   <213> Hepatitis B virus
<400> 10
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys
               180                 185
<210> 11
   <211> 21
   <212> DNA
   <213> Artificial Sequence
<220>
   <223> CyCpG
<400> 11
   tccatgacgt tcctgaataa t 21
<210> 12
   <211> 594
   <212> DNA
   <213> Artificial Sequence
<220>
   <223> Hepatitis B virus containing p33
<220>
   <221> CDS
   <222> (1)..(594)
<400> 12
   atg gac att gac cct tat aaa gaa ttt gga gct act gtg gag tta ctc      48
   Met Asp Ile Asp Pro Tyr Lys Glu Phe Gly Ala Thr Val Glu Leu Leu
   1               5                   10                  15
   tcg ttt ttg cct tct gac ttc ttt cct tcc gtc aga gat ctc cta gac      96
   Ser Phe Leu Pro Ser Asp Phe Phe Pro Ser Val Arg Asp Leu Leu Asp
               20                  25                  30
   acc gcc tca gct ctg tat cga gaa gcc tta gag tct cct gag cat tgc     144
   Thr Ala Ser Ala Leu Tyr Arg Glu Ala Leu Glu Ser Pro Glu His Cys
           35                  40                  45
   tca cct cac cat act gca ctc agg caa gcc att ctc tgc tgg ggg gaa      192
   Ser Pro His His Thr Ala Leu Arg Gln Ala Ile Leu Cys Trp Gly Glu
       50                  55                  60
   ttg atg act cta gct acc tgg gtg ggt aat aat ttg gaa gat cca gca      240
   Leu Met Thr Leu Ala Thr Trp Val Gly Asn Asn Leu Glu Asp Pro Ala
   65                  70                  75                  80
   tcc agg gat cta gta gtc aat tat gtt aat act aac atg ggt tta aag      288
   Ser Arg Asp Leu Val Val Asn Tyr Val Asn Thr Asn Met Gly Leu Lys
                   85                  90                  95
   atc agg caa cta ttg tgg ttt cat ata tct tgc ctt act ttt gga aga      336
   Ile Arg Gln Leu Leu Trp Phe His Ile Ser Cys Leu Thr Phe Gly Arg
               100                 105                 110
   gag act gta ctt gaa tat ttg gtc tct ttc gga gtg tgg att cgc act      384
   Glu Thr Val Leu Glu Tyr Leu Val Ser Phe Gly Val Trp Ile Arg Thr
           115                 120                 125
   cct cca gcc tat aga cca cca aat gcc cct atc tta tca aca ctt ccg      432
   Pro Pro Ala Tyr Arg Pro Pro Asn Ala Pro Ile Leu Ser Thr Leu Pro
       130                 135                 140
   gaa act act gtt gtt aga cga cgg gac cga ggc agg tcc cct aga aga      480
   Glu Thr Thr Val Val Arg Arg Arg Asp Arg Gly Arg Ser Pro Arg Arg
   145                 150                 155                 160
   aga act ccc tcg cct cgc aga cgc aga tct caa tcg ccg cgt cgc aga      528
   Arg Thr Pro Ser Pro Arg Arg Arg Arg Ser Gln Ser Pro Arg Arg Arg
                   165                 170                 175
   aga tct caa tct cgg gaa tct caa tgt ctt ctc ctt aaa gct gtt tac      576
   Arg Ser Gln Ser Arg Glu Ser Gln Cys Leu Leu Leu Lys Ala Val Tyr
               180                 185                 190
   aac ttc gct acc atg taa                                              594
   Asn Phe Ala Thr Met
           195
<210> 13
   <211> 197
   <212> PRT
   <213> Artificial Sequence
<220>
   <223> Hepatitis B virus containing p33
<400> 13
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ser Ala Leu Tyr Arg Glu Ala Leu Glu Ser Pro Glu His Cys
           35                  40                  45
   Ser Pro His His Thr Ala Leu Arg Gln Ala Ile Leu Cys Trp Gly Glu
       50                  55                  60
   Leu Met Thr Leu Ala Thr Trp Val Gly Asn Asn Leu Glu Asp Pro Ala
   65                  70                  75                  80
   Ser Arg Asp Leu Val Val Asn Tyr Val Asn Thr Asn Met Gly Leu Lys
                   85                  90                  95
   Ile Arg Gln Leu Leu Trp Phe His Ile Ser Cys Leu Thr Phe Gly Arg
               100                 105                 110
   Glu Thr Val Leu Glu Tyr Leu Val Ser Phe Gly Val Trp Ile Arg Thr
           115                 120                 125
   Pro Pro Ala Tyr Arg Pro Pro Asn Ala Pro Ile Leu Ser Thr Leu Pro
       130                 135                 140
   Glu Thr Thr Val Val Arg Arg Arg Asp Arg Gly Arg Ser Pro Arg Arg
   145                 150                 155                 160
   Arg Thr Pro Ser Pro Arg Arg Arg Arg Ser Gln Ser Pro Arg Arg Arg
                   165                 170                 175
   Arg Ser Gln Ser Arg Glu Ser Gln Cys Leu Leu Leu Lys Ala Val Tyr
               180                 185                 190
   Asn Phe Ala Thr Met
           195
<210> 14
   <211> 9
   <212> PRT
   <213> Homo sapiens
<400> 14
   Lys Thr Trp Gly Gln Tyr Trp Gln Val
   1 5
<210> 15
   <211> 9
   <212> PRT
   <213> Homo sapiens
<400> 15
   Ile Thr Asp Gln Val Pro Phe Ser Val
   1 5
<210> 16
   <211> 9
   <212> PRT
   <213> Homo sapiens
<400> 16
   Tyr Leu Glu Pro Gly Pro Val Thr Ala
   1 5
<210> 17
   <211> 10
   <212> PRT
   <213> Homo sapiens
<400> 17
   Leu Leu Asp Gly Thr Ala Thr Leu Arg Leu
   1 5 10
<210> 18
   <211> 10
   <212> PRT
   <213> Homo sapiens
<400> 18
   Val Leu Tyr Arg Tyr Gly Ser Phe Ser Val
   1 5 10
<210> 19
   <211> 9
   <212> PRT
   <213> Homo sapiens
<400> 19
   Ala Ala Gly Ile Gly Ile Leu Thr Val
   1 5
<210> 20
   <211> 9
   <212> PRT
   <213> Homo sapiens
<400> 20
   Ile Leu Thr Val Ile Leu Gly Val Leu
   1 5
<210> 21
   <211> 9
   <212> PRT
   <213> Homo sapiens
<400> 21
   Met Leu Leu Ala Val Leu Tyr Cys Leu
   1 5
<210> 22
   <211> 9
   <212> PRT
   <213> Homo sapiens
<400> 22
   Tyr Met Asp Gly Thr Met Ser Gln Val
   1 5
<210> 23
   <211> 9
   <212> PRT
   <213> Homo sapiens
<400> 23
   Val Leu Pro Asp Val Phe Ile Arg Cys
   1 5
<210> 24
   <211> 9
   <212> PRT
   <213> Homo sapiens
<400> 24
   Phe Leu Trp Gly Pro Arg Ala Leu Val
   1 5
<210> 25
   <211> 9
   <212> PRT
   <213> Homo sapiens
<400> 25
   Tyr Leu Ser Gly Ala Asn Leu Asn Leu
   1 5
<210> 26
   <211> 9
   <212> PRT
   <213> Homo sapiens
<400> 26
   Arg Met Pro Glu Ala Ala Pro Pro Val
   1 5
<210> 27
   <211> 9
   <212> PRT
   <213> Homo sapiens
<400> 27
   Ser Thr Pro Pro Pro Gly Thr Arg Val
   1 5
<210> 28
   <211> 9
   <212> PRT
   <213> Homo sapiens
<400> 28
   Leu Leu Gly Arg Asn Ser Phe Glu Val
   1 5
<210> 29
   <211> 9
   <212> PRT
   <213> Homo sapiens
<400> 29
   Lys Ile Phe Gly Ser Leu Ala Phe Leu
   1 5
<210> 30
   <211> 9
   <212> PRT
   <213> Homo sapiens
<400> 30
   Ile Ile Ser Ala Val Val Gly Ile Leu
   1 5
<210> 31
   <211> 8
   <212> PRT
   <213> Homo sapiens
<400> 31
   Thr Leu Gly Ile Val Cys Pro Ile
   1 5
<210> 32
   <211> 131
   <212> PRT
   <213> Bacteriophage AP205
<400> 32
   Met Ala Asn Lys Pro Met Gln Pro Ile Thr Ser Thr Ala Asn Lys Ile
   1 5 10 15
   Val Trp Ser Asp Pro Thr Arg Leu Ser Thr Thr Phe Ser Ala Ser Leu
   20 25 30
   Leu Arg Gln Arg Val Lys Val Gly Ile Ala Glu Leu Asn Asn Val Ser
   35 40 45
   Gly Gln Tyr Val Ser Val Tyr Lys Arg Pro Ala Pro Lys Pro Glu Gly
   50 55 60
   Cys Ala Asp Ala Cys Val Ile Met Pro Asn Glu Asn Gln Ser Ile Arg
   65 70 75 80
   Thr Val Ile Ser Gly Ser Ala Glu Asn Leu Ala Thr Leu Lys Ala Glu
   85 90 95
   Trp Glu Thr His Lys Arg Asn Val Asp Thr Leu Phe Ala Ser Gly Asn
   100 105 110
   Ala Gly Leu Gly Phe Leu Asp Pro Thr Ala Ala Ile Val Ser Ser Asp
   115 120 125
   Thr Thr Ala
   130
<210> 33
   <211> 131
   <212> PRT
   <213> Artificial Sequence
<220>
   <223> Bacteriophage AP205 mutant
<400> 33
   Met Ala Asn Lys Thr Met Gln Pro Ile Thr Ser Thr Ala Asn Lys Ile
   1               5                   10                  15
   Val Trp Ser Asp Pro Thr Arg Leu Ser Thr Thr Phe Ser Ala Ser Leu
               20                  25                  30
   Leu Arg Gln Arg Val Lys Val Gly Ile Ala Glu Leu Asn Asn Val Ser
           35                  40                  45
   Gly Gln Tyr Val Ser Val Tyr Lys Arg Pro Ala Pro Lys Pro Glu Gly
       50                  55                  60
   Cys Ala Asp Ala Cys Val Ile Met Pro Asn Glu Asn Gln Ser Ile Arg
   65                  70                  75                  80
   Thr Val Ile Ser Gly Ser Ala Glu Asn Leu Ala Thr Leu Lys Ala Glu
                   85                  90                  95
   Trp Glu Thr His Lys Arg Asn Val Asp Thr Leu Phe Ala Ser Gly Asn
               100                 105                 110
   Ala Gly Leu Gly Phe Leu Asp Pro Thr Ala Ala Ile Val Ser Ser Asp
           115                 120                 125
   Thr Thr Ala
       130
<210> 34
   <211> 5
   <212> PRT
   <213> Artificial Sequence
<220>
   <223> HBcAg peptide
<400> 34
   Gly Gly Lys Gly Gly
   1               5
<210> 35
   <211> 152
   <212> PRT
   <213> Artificial Sequence
<220>
   <223> HBcAg variant
<400> 35
   Met Asp Ile Asp Pro Tyr Lys Glu Phe Gly Ala Thr Val Glu Leu Leu
   1               5                   10                  15
   Ser Phe Leu Pro Ser Asp Phe Phe Pro Ser Val Arg Asp Leu Leu Asp
               20                  25                  30
   Thr Ala Ala Ala Leu Tyr Arg Asp Ala Leu Glu Ser Pro Glu His Cys
           35                  40                  45
   Ser Pro His His Thr Ala Leu Arg Gln Ala Ile Leu Cys Trp Gly Asp
       50                  55                  60
   Leu Met Thr Leu Ala Thr Trp Val Gly Thr Asn Leu Glu Asp Gly Gly
   65                  70                  75                  80
   Lys Gly Gly Ser Arg Asp Leu Val Val Ser Tyr Val Asn Thr Asn Val
                   85                  90                  95
   Gly Leu Lys Phe Arg Gln Leu Leu Trp Phe His Ile Ser Cys Leu Thr
               100                 105                 110
   Phe Gly Arg Glu Thr Val Leu Glu Tyr Leu Val Ser Phe Gly Val Trp
           115                 120                 125
   Ile Arg Thr Pro Pro Ala Tyr Arg Pro Pro Asn Ala Pro Ile Leu Ser
       130                 135                 140
   Thr Leu Pro Glu Thr Thr Val Val
   145                 150
<210> 36
   <211> 185
   <212> PRT
   <213> Artificial Sequence
<220>
   <223> HBcAg variant
<400> 36
   Met Asp Ile Asp Pro Tyr Lys Glu Phe Gly Ala Thr Val Glu Leu Leu
   1 5 10 15
   Ser Phe Leu Pro Ser Asp Phe Phe Pro Ser Val Arg Asp Leu Leu Asp
   20 25 30
   Thr Ala Ser Ala Leu Tyr Arg Glu Ala Leu Glu Ser Pro Glu His Ser
   35 40 45
   Ser Pro His His Thr Ala Leu Arg Gln Ala Ile Leu Cys Trp Gly Glu
   50 55 60
   Leu Met Thr Leu Ala Thr Trp Val Gly Asn Asn Leu Glu Asp Pro Ala
   65 70 75 80
   Ser Arg Asp Leu Val Val Asn Tyr Val Asn Thr Asn Met Gly Leu Lys
   85 90 95
   Ile Arg Gln Leu Leu Trp Phe His Ile Ser Ser Leu Thr Phe Gly Arg
   100 105 110
   Glu Thr Val Leu Glu Tyr Leu Val Ser Phe Gly Val Trp Ile Arg Thr
   115 120 125
   Pro Pro Ala Tyr Arg Pro Pro Asn Ala Pro Ile Leu Ser Thr Leu Pro
   130 135 140
   Glu Thr Thr Val Val Arg Arg Arg Asp Arg Gly Arg Ser Pro Arg Arg
   145 150 155 160
   Arg Thr Pro Ser Pro Arg Arg Arg Arg Ser Gln Ser Pro Arg Arg Arg
   165 170 175
   Arg Ser Gln Ser Arg Glu Ser Gln Cys
   180 185
<210> 37
   <211> 10
   <212> PRT
   <213> Homo sapiens
<400> 37
   Glu Ala Ala Gly Ile Gly Ile Leu Thr Val
   1 5 10
<210> 38
   <211> 10
   <212> PRT
   <213> Homo sapiens
<400> 38
   Glu Leu Ala Gly Ile Gly Ile Cys Thr Val
   1 5 10
<210> 39
   <211> 10
   <212> DNA
   <213> Artificial Sequence
<220>
   <223> oligonucleotide ISS
<400> 39
   gacgatcgtc 10
<210> 40
   <211> 19
   <212> DNA
   <213> Artificial Sequence
<220>
   <223> oligonucleotide G3-6
<400> 40
   ggggacgatc gtcgggggg 19
<210> 41
   <211> 20
   <212> DNA
   <213> Artificial Sequence
<220>
   <223> oligonucleotide G4-6
<400> 41
   gggggacgat cgtcgggggg 20
<210> 42
   <211> 21
   <212> DNA
   <213> Artificial Sequence
<220>
   <223> oligonucleotide G5-6
<400> 42
   ggggggacga tcgtcggggg g 21
<210> 43
   <211> 22
   <212> DNA
   <213> Artificial sequence
<220>
   <223> oligonucleotide G6-6
<400> 43
   gggggggacg atcgtcgggg gg 22
<210> 44
   <211> 24
   <212> DNA
   <213> Artificial sequence
<220>
   <223> oligonucleotide G7-7
<400> 44
   ggggggggac gatcgtcggg gggg 24
<210> 45
   <211> 26
   <212> DNA
   <213> Artificial sequence
<220>
   <223> oligonucleotide G8-8
<400> 45
   ggggggggga cgatcgtcgg gggggg 26
<210> 46
   <211> 28
   <212> DNA
   <213> Artificial Sequence
<220>
   <223> oligonucleotide G9-9
<400> 46
   gggggggggg acgatcgtcg gggggggg 28
<210> 47
   <211> 30
   <212> DNA
   <213> Artificial sequence
<220>
   <223> oligonucleotide G6
<400> 47
   ggggggcgac gacgatcgtc gtcggggggg 30
<210> 48
   <211> 23
   <212> DNA
   <213> Artificial Sequence
<220>
   <223> CpG-2006, deoxynucleotides connected via phosphorothioate bonds
<400> 48
   tcgtcgtttt gtcgttttgt cgt 23
<210> 49
   <211> 21
   <212> DNA
   <213> Artificial Sequence
<220>
   <223> CyCpGpt, deoxynucleotides connected via phosphorothioate bonds
<400> 49
   tccatgacgt tcctgaataa t 21
<210> 50
   <211> 20
   <212> DNA
   <213> Artificial Sequence
<220>
   <223> B-CpGpt, deoxynucleotides connected via phosphorothioate bonds
<400> 50
   tccatgacgt tcctgacgtt 20
<210> 51
   <211> 20
   <212> DNA
   <213> Artificial Sequence
<220>
   <223> B-CpG
<400> 51
   tccatgacgt tcctgacgtt 20
<210> 52
   <211> 19
   <212> DNA
   <213> Artificial Sequence
<220>
   <223> NKCpG
<400> 52
   ggggtcaacg ttgaggggg 19
<210> 53
   <211> 21
   <212> DNA
   <213> Artificial Sequence
<220>
   <223> CyCpG-rev-pt, deoxynucleotides connected via phosphorothioate bonds
<400> 53
   attattcagg aacgtcatgg a 21
<210> 54
   <211> 30
   <212> DNA
   <213> Artificial Sequence
<220>
   <223> g10gacga-PO (G10-PO)
<400> 54
   gggggggggg gacgatcgtc gggggggggg 30
<210> 55
   <211> 30
   <212> DNA
   <213> Artificial Sequence
<220>
   <223> g10gacga-PS (G10-PS), deoxynucleotides connected via phosphorothioate bonds
<400> 55
   gggggggggg gacgatcgtc gggggggggg 30
<210> 56
   <211> 62
   <212> DNA
   <213> Artificial Sequence
<220>
   <223> (CpG)200pA
<400> 56
   cgcgcgcgcg cgcgcgcgcg cgcgcgcgcg cgcgcgcgcg aaatgcatgt caaagacagc 60
   at 62
<210> 57
   <211> 61
   <212> DNA
   <213> Artificial Sequence
<220>
   <223> Cy(CpG)20
<400> 57
   tccatgacgt tcctgaataa tcgcgcgcgc gcgcgcgcgc gcgcgcgcgc gcgcgcgcgc 60
   g 61
<210> 58
   <211> 83
   <212> DNA
   <213> Artificial Sequence
<220>
   <223> Cy(CpG)20-OpA
<400> 58
   tccatgacgt tcctgaataa tcgcgcgcgc gcgcgcgcgc gcgcgcgcgc gcgcgcgcgc 60
   gaaatgcatg tcaaagacag cat 83
<210> 59
   <211> 253
   <212> DNA
   <213> Artificial Sequence
<220>
   <223> dsCyCpG-253
<400> 59
   ctagaactag tggatccccc gggctgcagg aattcgattc atgacttcct gaataattcc 60
   atgacgttgg tgaataattc catgacgttc ctgaataatt ccatgacgtt cctgaataat 120
   tccatgacgt tcctgaataa ttccatgacg ttcctgaata attccatgac gttcctgaat 180
   aattccatga cgttcctgaa taattccatg acgttcctga aaattccaat caagcttatc 240
   gataccgtcg acc 253
<210> 60
   <211> 9
   <212> PRT
   <213> homo sapiens
<400> 60
   Lys Ala Val Tyr Asn Phe Ala Thr Met
   1 5

## Description

### BACKGROUND OF THE INVENTION

### Field of the Invention

The present invention is related to the fields of vaccinology, immunology and medicine. The invention provides compositions for enhancing immunological responses against antigens coupled to virus-like particles (VLPs) packaged with immunostimulatory nucleic acids, namely oligonucleotides containing at least one non-methylated CpG sequence and a toll-like receptor (TLR) -4 ligand. The invention can be used to induce strong antibody and T cell responses particularly useful for the treatment of allergies, tumors and chronic viral diseases as well as other chronic diseases.

### Related Art

It is usually difficult to induce antibody responses against self-antigens. One way to improve the efficiency of vaccination is to increase the degree of repetitiveness of the antigen applied. Unlike isolated proteins, viruses induce prompt and efficient immune responses in the absence of any adjuvant both with and without T-cell help (Bachmann and Zinkemagel, Ann. Rev. Immunol. 15:235-270 (1991)). Although viruses often consist of few proteins, they are able to trigger much stronger immune responses than their isolated components. For B-cell responses, it is known that one crucial factor for the immunogenicity of viruses is the repetitiveness and order of surface epitopes. Many viruses exhibit a quasi-crystalline surface that displays a regular array of epitopes which efficiently crosslinks epitope-specific immunoglobulins on B-cells (Bachmann and Zinkernagel, Immunol. Today 17:553-558 (1996)). This crosslinking of surface immunoglobulins on B cells is a strong activation signal that directly induces cell-cycle progression and the production of IgM antibodies. Further, such triggered B-cells are able to activate T helper cells, which in turn induce a switch from IgM to IgG antibody production in B cells and the generation of long-lived B cell memory - the goal of any vaccination (Bachmann and Zinkernagel, Ann. Rev. Immunol. 15:235-270 (1997)). Viral structure is even linked to the generation of anti-antibodies in autoimmune disease and as a part of the natural response to pathogens (see Fehr, T., et al., J Exp. Med. 185:1785-1792 (1997)). Thus, antigens presented by a highly organized viral surface are able to induce strong antibody responses against the antigens

As indicated, however, the immune system usually fails to produce antibodies against self-derived structures. For soluble antigens present at low concentrations, this is due to tolerance at the Th-cell level. Under these conditions, coupling the self-antigen to a carrier that can deliver T help may break tolerance. For soluble proteins present at high concentrations or membrane proteins at low concentration, B- and Th-cells may be tolerant. However, B-cell tolerance may be reversible (anergy) and can be broken by administration of the antigen in a highly organized fashion coupled to a foreign carrier (Bachmann and Zinkemagel, Ann. Rev. Immunol. 15:235-270 (1997)).

Recent evidence demonstrates that virus-like particles (VLPs) containing packaged CpGs are able to trigger T cell responses against antigens conjugated to the VLPs (WO03/024481). In addition, packaging CpGs enhanced their stability and essentially removed their above mentioned side-effects such as causing extramedullary hemopoiesis accompanied by splenomegaly and lymphadenopathy in mice.

In contrast to CpGs, which engange TLR9 on APCs, other TLR-ligands alone failed to enhance VLP-induced T cell responses (Schwarz et al., (2003) Eur. J. Immunol., 33, 1465-1470). Specifically, peptidoglycans, a ligand for TLR2, poly (I:C), a ligand for TLR3, LPS, a ligand for TLR4, flagellin, a ligand for TLR5 and imiquimode, a ligand for TLR7 all failed to enhance VLP-induced CTL responses in a way similar to CpGs.

There have been remarkable advances made in vaccination strategies recently, yet there remains a need for improvement on existing strategies. In particular, there remains a need in the art for the development of new and improved vaccines that allow the induction of strong T and B cell responses without serious side-effects and, in particular, that allow the promotion of a strong CTL immune response and anti-pathogenic protection as efficiently as natural pathogens.

### SUMMARY OF THE INVENTION

This invention is based on the surprising finding that immunostimulatory nucleic acids, namely DNA oligonucleotides containing CpG motifs which stimulate Toll-like receptor 9 (TLR9), packaged into VLPs enhance B and T cell responses to antigens coupled to VLPs whereas other ligands for TLRs, including peptidoglycans, a ligand for TLR2, poly (I:C), a ligand for TLR3, LPS, a ligand for TLR4, flagellin, a ligand for TLR5 and imiquimode, a ligand for TLR7 all failed to enhance VLP-induced T responses in a way similar to CpGs (Schwarz et al., (2003) Eur. J. Immunol., 33, 1465-1470). Surprisingly, however, although ligands for TLRs other than TLR9, such as e.g. TLR4, failed to enhance T cell responses against antigens coupled or fused to VLPs, they efficiently enhanced T cell responses in the presence of immunostimulatory nucleic acids, in particular unmethylated CpG-containing oligonucleotides. Thus, there was synergistic effect between ligands for TLR 4 and unmethylated CpG containing oligonucleotides.

In one aspect, the invention relates to a composition for enhancing an immune response in an animal comprising: (a) a virus-like particle; (b) an immunostimulatory DNA oligonucleotide which stimulates TLR9, wherein said DNA oligonucleotide is an unmethylated CpG-containing oligonucleotide packaged into said virus-like particle (a); (c) at least one antigen, wherein said antigen is coupled to said virus-like particle (a); and (d) at least one toll-like receptor (TLR) ligand activating a TLR 4, wherein said ligand is selected from the group consisting of: LPS and derivatives thereof, Monosphoryl lipid A and derivatives thereof, and synthetic analoga of LPS.

Also disclosed is a composition for enhancing an immune response in an animal comprising (a) a VLP, (b) an immunostimulatory nucleic acid, preferably an unmethylated CpG-containing oligonucleotide, where the nucleic acid or oligonucleotide is mixed with the virus-like particle, (c) at least one antigen coupled or fused to the VLP or an antigen mixed with the virus-like particle, and (d) at least one ligand for a TLR.

Immunostimulatory nucleic acids which do not contain CpG motifs but nevertheless exhibit immunostimulatory activities are described in WO 01/22972.

In a preferred embodiment of the invention, the unmethylated CpG-containing oligonucleotide is not stabilized by phosphorothioate modifications of the phosphodiester backbone.

In a preferred embodiment, the unmethylated CpG containig oligonucleotide induces IFN-alpha in human cells. In another preferred embodiment, the IFN-alpha inducing oligonucleotide is flanked by guanosine-rich repeats and contains a palindromic sequence.

In a further preferred embodiment, the virus-like particle is a recombinant virus-like particle. Also preferred, the virus-like particle is free of a lipoprotein envelope. Further disclosed is a recombinant virus-like particle which comprises, or alternatively consists of, recombinant proteins of Hepatitis B virus, measles virus, Sindbis virus, Rotavirus, Foot-and-Mouth-Disease virus, Retrovirus, Norwalk virus or human Papilloma virus RNA-phages, Qβ-phage, GA-phage, fr-phage, AP205-phage and Ty. Further disclosed is a virus-like particle which comprises, or alternatively consists of, one or more different Hepatitis B virus core (capsid) proteins (HBcAgs).

In a further preferred embodiment, the virus-like particle comprises recombinant proteins, or fragments thereof, of a RNA-phage. Preferred RNA-phages are Qβ-phage, AP 205-phage, GA-phage, fr-phage.

The antigen, antigens or antigen mixture may be a recombinant antigen. The antigen, antigens or antigen mixture may be extracted from a natural source, which includes but is not limited to: pollen, dust, fungi, insects, food, mammalian epidermals, hair, saliva, serum, bees, tumors, pathogens and feathers.

The antigen can be selected from the group consisting of: (1) a polypeptide suited to induce an immune response against cancer cells; (2) a polypeptide suited to induce an immune response against infectious diseases; (3) a polypeptide suited to induce an immune response against allergens; (4) a polypeptide suited to induce an improved response against self-antigens; and (5) a polypeptide suited to induce an immune response in farm animals or pets.

The antigen, antigens or antigen mixture can be selected from the group consisting of: (1) an organic molecule suited to induce an immune response against cancer cells; (2) an organic molecule suited to induce an immune response against infectious diseases; (3) an organic molecule suited to induce an immune response against allergens; (4) an organic molecule suited to induce an improved response against self-antigens; (5) an organic molecule suited to induce an immune response in farm animals or pets; and (6) an organic molecule suited to induce a response against a drug, a hormone or a toxic compound.

The antigen may comprise, or alternatively consists of, a cytotoxic T cell or Th cell epitope. In a related embodiment, the antigen comprises or alternatively consists of, a B cell epitope. Also discloses is a virus like particle which comprises the Hepatitis B virus core protein

The additional ligand for TLRs added to the virus-like particle loaded with CpGs is recognized by TLR4. Such a ligand may be LPS or, preferably, a detoxified version of LPS, such as MPL (Nat Biotechnol 17: 1075) or synthetic ligands for TLR4.

Further disclosed is a method of enhancing an immune response in a human or other animal species comprising introducing into the animal a composition comprising (a) a VLP, (b) an immunostimulatory nucleic acid, preferably an unmethylated CpG-containing oligonucleotide, where the nucleic acid or oligonucleotide is mixed with, coupled to, fused to, or otherwise attached to or enclosed by, *i.e*., bound to, and preferably packaged with the virus-like particle, (c) at least one antigen coupled or fused to the VLP or an antigen mixed with the virus-like particle, and (d) at least one ligand for a TLR.

The composition may be introduced into an animal subcutaneously, intramuscularly, intranasally, intradermally, intravenously or directly into a lymph node. The immune enhancing composition may be applied locally, near a tumor or local viral reservoir against which one would like to vaccinate.

The immune response may be a T cell response, and the T cell response against the antigen is enhanced, whereby the T cell response may be a cytotoxic T cell response, and the cytotoxic T cell response against the antigen is enhanced. Also, the immune response may be a B cell response, and the B cell response against the antigen is enhanced.

Also disclosed is a vaccine comprising an immunologically effective amount of the immune enhancing composition of the present invention together with a pharmaceutically acceptable diluent, carrier or excipient. Further disclosed is a method of immunizing and/or treating an animal comprising administering to the animal an immunologically effective amount of the disclosed vaccine.

The immunostimulatory nucleic acid-containing VLP's, namely the unmethylated CpG-containing oligonucleotide VLPs are used for vaccination of animals or humans against antigens coupled to the modified VLP. The modified VLPs can be used to vaccinate against tumors, viral diseases, or self-molecules, for example. The vaccination can be for prophylactic or therapeutic purposes, or both. Also, the modified VLPs can be used to vaccinate against allergies, or diseases related to allergy such as asthma, in order to induce immune-deviation and/or antibody responses against the allergen. Such a vaccination and treatment, respectively, can then lead, for example, to a desensibilization of a former allergic animal and patient, respectively.

In the majority of cases, the desired immune response will be directed against antigens coupled to or mixed with the immunostimulatory nucleic acid-containing VLPs, preferably the unmethylated CpG-containing oligonucleotide VLPs. The antigens can be peptides, proteins or domains as well as mixtures thereof

The route of injection is preferably subcutaneous or intramuscular, but it would also be possible to apply the CpG-containing VLPs intradermally, intranasally, intravenously or directly into the lymph node. In an equally preferred embodiment, the CpG-containing VLPs mixed or coupled with antigen are applied locally, near a tumor or local viral reservoir against which one would like to vaccinate.

It is to be understood that both the foregoing general description and the following detailed description are exemplary and explanatory only and are intended to provide further explanation of the invention as claimed.

### BRIEF DESCRIPTION OF THE DRAWINGS/FIGURES

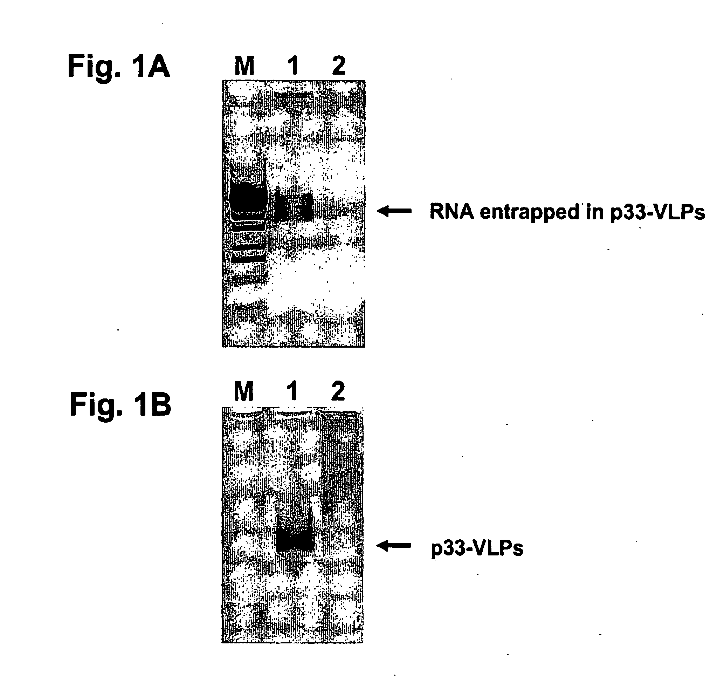

Fig. 1 shows VLPs in a native agarose gel electrophoresis (1% agarose) after control incubation or after digestion with RNase A upon staining with ethidium bromide (A) or Coomassie blue (B) in order to assess for the presence of RNA or protein. Recombinantly produced VLPs were diluted at a final concentration of 0.5 ug/ul protein in PBS buffer and incubated in the absence (lane 1) or presence (lane 2) of RNase A (100 ug/ml) (Sigma, Division of Fluka AG, Switzerland) for 2 h at 37°C. The samples were subsequently complemented with 6-fold concentrated DNA-loading buffer (MBS Fermentas GmbH, Heidelberg, Germany) and run for 30 min at 100 volts in a 1% native agarose gel. The Gene Ruler marker (MBS Fermentas GmbH, Heidelberg, Germany) was used as reference for VLPs migration velocity (lane M). Rows are indicating the presence of RNA enclosed in VLPs (A) or VLPs itself (B). Identical results were obtained in 3 independent experiments.

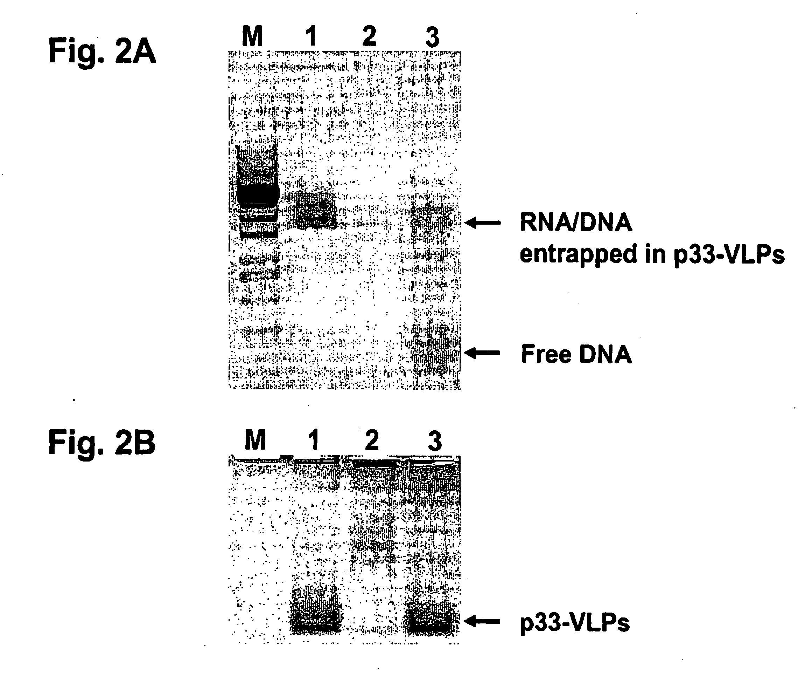

Fig. 2 shows VLPs in a native agarose gel electrophoresis (1% agarose) after control incubation or after digestion with RNase A in the presence of buffer only or CpG-containing DNA-oligonucleotides upon staining with ethidium bromide (A) or Comassie blue (B) in order to assess for the presence of RNA/DNA or protein. Recombinant VLPs were diluted at a final concentration of 0.5 ug/ul protein in PBS buffer and incubated in the absence (lane 1) or presence (lane 2 and 3) of RNase A (100 ug/ml) (Sigma, Division of Fluka AG, Switzerland) for 2 h at 37°C. 5 nmol CpG-oligonucleotides (containing phosphorothioate modifications of the phosphate backbone) were added to sample 3 before RNase A digestion. The Gene Ruler marker (MBS Fermentas GmbH, Heidelberg, Germany) was used as reference for p33-VLPs migration velocity (lane M). Rows are indicating the presence of RNA/CpG-DNA enclosed in p33-VLPs (A) or p33-VLPs itself (B). Comparable results were obtained when CpG oligonucleotides with normal phosphor bonds were used for co-incubation of VLPs with RNase A.

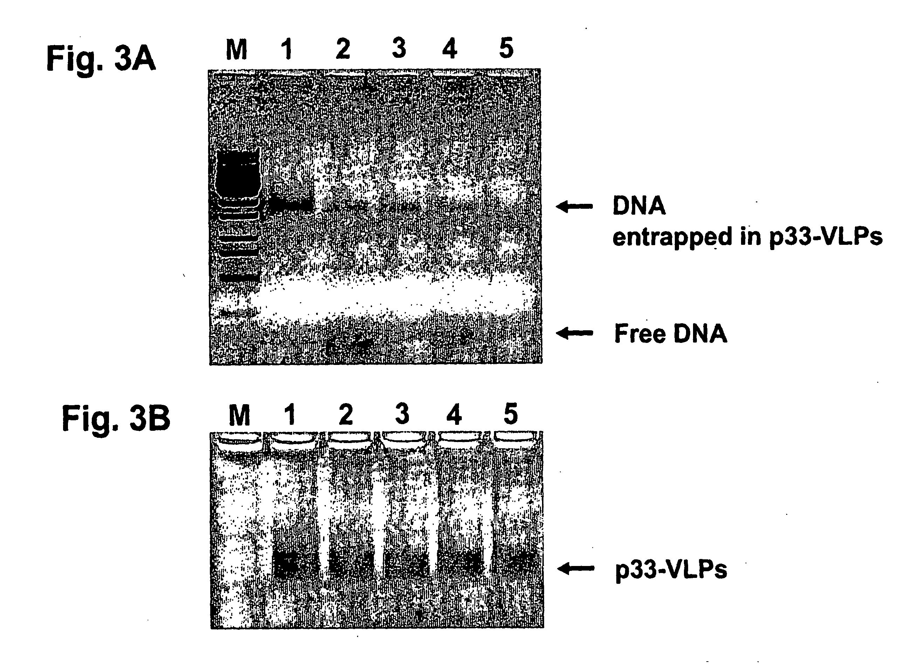

Fig. 3 shows p33-VLPs in a native agarose gel electrophoresis (1% agarose) before and after digestion with RNase A in the presence of CpG-containing DNA-oligonucleotides and subsequent dialysis (for the elimination of VLP-unbound CpG-oligonucleotides) upon staining with ethidium bromide (A) or Comassie blue (B) in order to assess for the presence of DNA or protein. Recombinant VLPs were diluted at a final concentration of 0.5 ug/ul protein in PBS buffer and incubated in absence (lane 1) or in presence (lanes 2 to 5) of RNase A (100 ug/ml) (Sigma, Division of Fluka AG, Switzerland) for 2 h at 37°C. 50 nmol CpG-oligonucleotides (containing phosphorothioate bonds: lanes 2 and 3, containing normal phosphor modifications of the phosphate backbone: lanes 4 and 5) were added to VLPs before RNase A digestion. Treated samples were extensively dialysed for 24 hours against PBS (4500-fold dilution) with a 300 kDa MWCO dialysis membrane (Spectrum Medical Industries Inc., Houston, USA) to eliminate the in excess DNA (lanes 3 and 5). The Gene Ruler marker (MBS Fermentas GmbH, Heidelberg, Germany) was used as reference for p33-VLPs migration velocity (lane M). Rows are indicating the presence of RNA/CpG-DNA enclosed in VLPs (A) or VLPs itself (B).

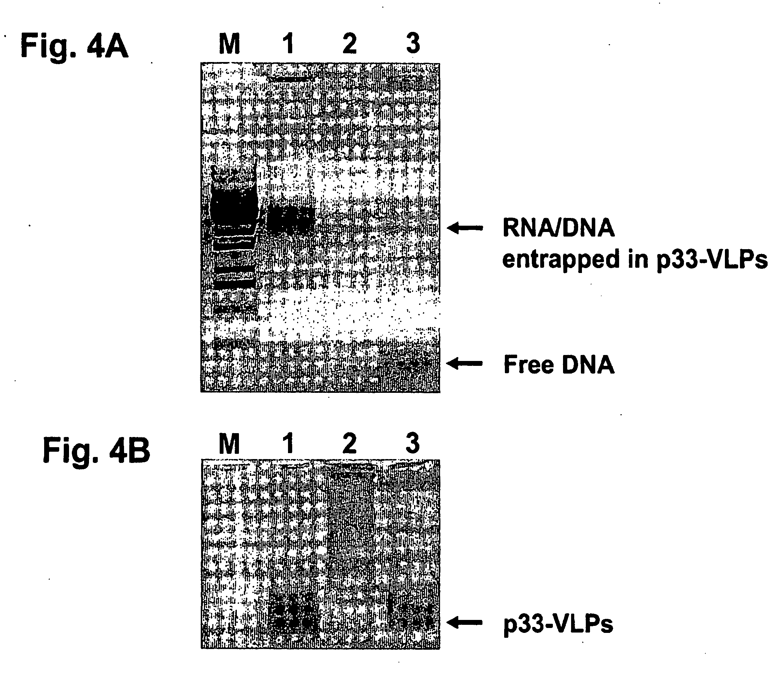

Fig. 4 shows VLPs in a native agarose gel electrophoresis (1% agarose) after control incubation or after digestion with RNase A where CpG-containing DNA-oligonucleotides were added only after completing the RNA digestion upon staining with ethidium bromide (A) or Comassie blue (B) in order to assess for the presence of RNA/DNA or protein. Recombinant VLPs were diluted at a final concentration of 0.5 ug/ul protein in PBS buffer and incubated in the absence (lane 1) or presence (lane 2 and 3) of RNase A (100 ug/ml) (Sigma, Division of Fluka AG, Switzerland) for 2 h at 37°C. 5 nmol CpG-oligonucleotides (containing phosphorothioate modifications of the phosphate backbone) were added to sample 3 only after the RNase A digestion. The Gene Ruler marker (MBS Fermentas GmbH, Heidelberg, Germany) was used as reference for p33-VLPs migration velocity (lane M). Rows are indicating the presence of RNA/CpG-DNA enclosed in VLPs (A) or VLPs itself (B). Similar results were obtained when CpG oligonucleotides with normal phosphor bonds were used for reassembly of VLPs.

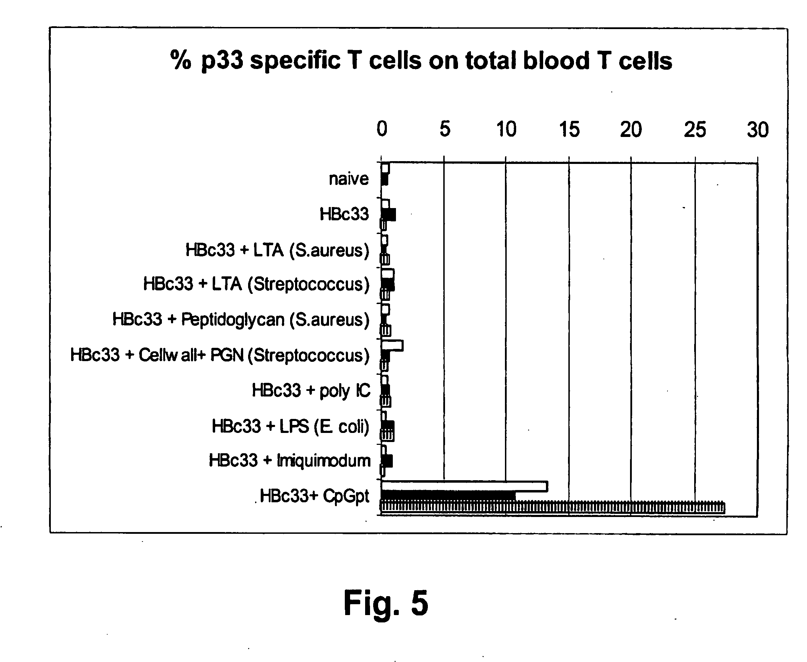

Fig. 5 shows that various ligands for TLRs, with the exception of the TLR9 ligand CpGs, fail to enhance the T cell response against peptide p33 fused to the hepatis B core antigen (p33-VLPs). Mice were immunized with p33-VLPs in the presence of PBS or the indicated stimuli of TLRs. 100 ug HBc33 and 100 ug adjuvant were used. Frequencies of p33-specific T cells was assessed 8 days later by tetramer staining. Each bar representd one individual mouse. (LTA = Lipoteichonic acid, PGN = Peptidoglycan, LPS from E. coli K-235, Sigma).

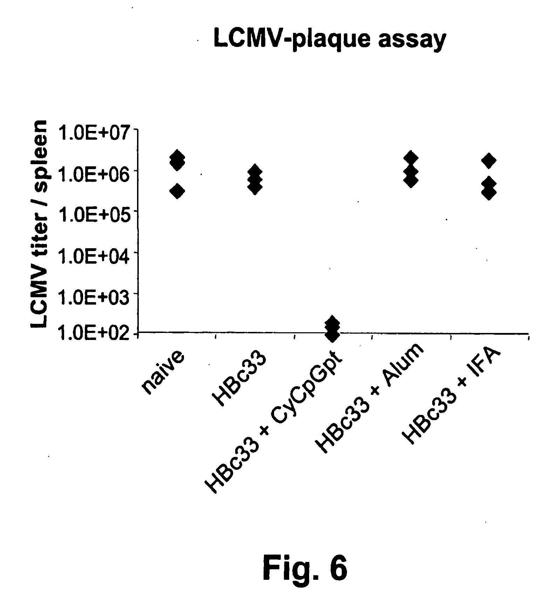

Fig. 6 shows that the prototype adjuvants Alum and IFA fail to enhance VLP-induced immunity. Mice were vaccinested with p33-VLPs in the presence of PBS, CpGs, Alum or IFA and challenged 8 days later with live LCMV (200 pfu). Viral titers were determined 5 days later in the spleen.

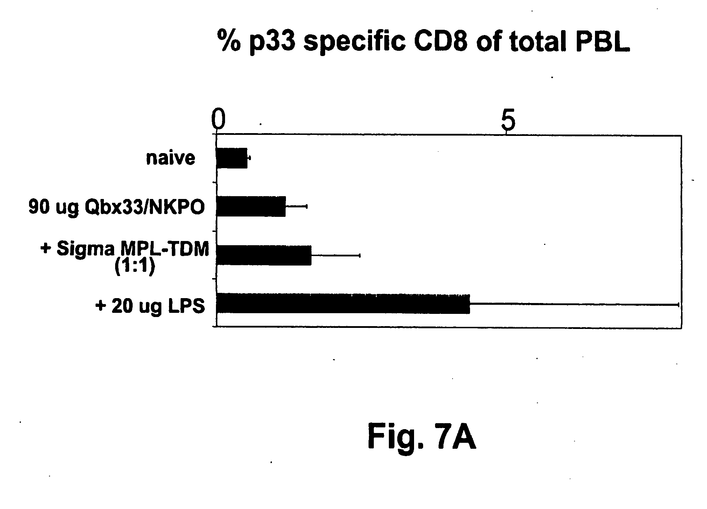

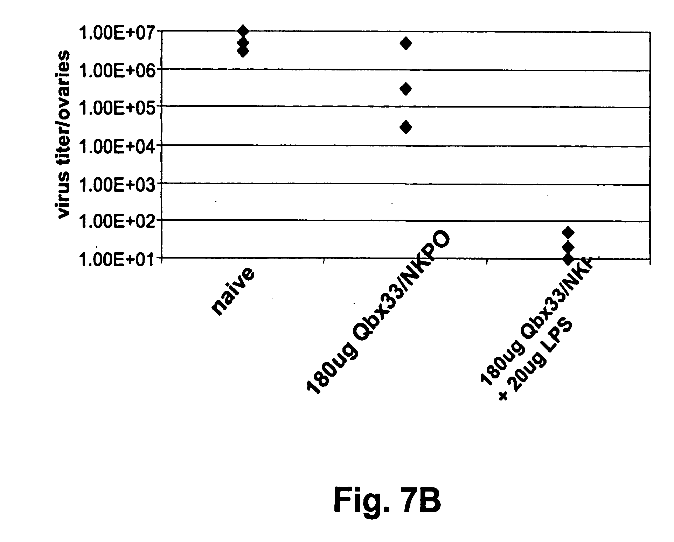

Fig. 7 shows that ligands for TLR4 enhance CTL response against p33 coupled to VLPs loaded with CpGs. Mice were vaccinated with p33 coupled to Qb loaded with NK-PO CpGs in the presence of PBS, LPS or MPL (1:1 mixture). Eight days later, frequencies of p33-specific T cells were assessed by tetramer staining (A) On the same day, mice were challenged with recmombinant vaccina virus expressing LCMV-GP and viral titers were determined 5 days later in ovaries (B).

### DETAILED DESCRIPTION OF THE INVENTION

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods and materials are hereinafter described.

### 1. Definitions

Amino acid linker: An "amino acid linker", or also just termed "linker" within this specification, as used herein, either associates the antigen or antigenic determinant with the second attachment site, or more preferably, already comprises or contains the second attachment site, typically - but not necessarily - as one amino acid residue, preferably as a cysteine residue. The term "amino acid linker" as used herein, however; does not intend to imply that such an amino acid linker consists exclusively of amino acid residues, even if an amino acid linker consisting of amino acid residues is a preferred embodiment of the present invention. The amino acid residues of the amino acid linker are, preferably, composed of naturally occuring amino acids or unnatural amino acids known in the art, all-L or all-D or mixtures thereof. However, an amino acid linker comprising a molecule with a sulfhydryl group or cysteine residue is also encompassed within the invention. Such a molecule comprise preferably a C1-C6 alkyl-, cycloalkyl (C5,C6), aryl or heteroaryl moiety. However, in addition to an amino acid linker, a linker comprising preferably a C1-C6 alkyl-, cycloalkyl- (C5,C6), aryl- or heteroaryl- moiety and devoid of any amino acid(s) shall also be encompassed within the scope of the invention. Association between the antigen or antigenic determinant or optionally the second attachment site and the amino acid linker is preferably by way of at least one covalent bond, more preferably by way of at least one peptide bond.

Animal: As used herein, the term "animal" is meant to include, for example, humans, sheep, horses, cattle, pigs, dogs, cats, rats, mice, birds, reptiles, fish, insects and arachnids.

Antibody: As used herein, the term "antibody" refers to molecules which are capable of binding an epitope or antigenic determinant. The term is meant to include whole antibodies and antigen-binding fragments thereof, including single-chain antibodies. Most preferably the antibodies are human antigen binding antibody fragments and include, but are not limited to, Fab, Fab' and F(ab')2, Fd, single-chain Fvs (scFv), single-chain antibodies, disulfide-linked Fvs (sdFv) and fragments comprising either a V_{L} or V_{H} domain. The antibodies can be from any animal origin including birds and mammals. Preferably, the antibodies are human, murine, rabbit, goat, guinea pig, camel, horse or chicken. As used herein, "human" antibodies include antibodies having the amino acid sequence of a human immunoglobulin and include antibodies isolated from human immunoglobulin libraries or from animals transgenic for one or more human immunoglobulins and that do not express endogenous immunoglobulins, as described, for example, in U.S. Patent No. 5,939,598 by Kucherlapati et al.

In a preferred embodiment of the invention, compositions of the invention may be used in the design of vaccines for the treatment of allergies. Antibodies of the IgE isotype are important components in allergic reactions. Mast cells bind IgE antibodies on their surface and release histamines and other mediators of allergic response upon binding of specific antigen to the IgE molecules bound on the mast cell surface. Inhibiting production of IgE antibodies, therefore, is a promising target to protect against allergies. This should be possible by attaining a desired T helper cell response. T helper cell responses can be divided into type 1 (T_{H}1) and type 2 (T_{H}2) T helper cell responses (Romagnani, Immunol. Today 18:263-266 (1997)). T_{H}1 cells secrete interferon-gamma and other cytokines which trigger B cells to produce IgG antibodies. In contrast, a critical cytokine produced by T_{H}2 cells is IL-4, which drives B cells to produce IgE. In many experimental systems, the development of T_{H}1 and T_{H}2 responses is mutually exclusive since T_{H}1 cells suppress the induction of T_{H}2 cells and vice versa. Thus, antigens that trigger a strong T_{H}1 response simultaneously suppress the development of T_{H}2 responses and hence the production of IgE antibodies. The presence of high concentrations of IgG antibodies may prevent binding of allergens to mast cell bound IgE, thereby inhibiting the release of histamine. Thus, presence of IgG antibodies may protect from IgE mediated allergic reactions. Typical substances causing allergies include, but are not limited to: pollens (*e.g*. grass, ragweed, birch or mountain cedar); house dust and dust mites; mammalian epidermal allergens and animal danders; mold and fungus; insect bodies and insect venom; feathers; food; and drugs (*e.g*., penicillin). See Shough, H. et al., REMINGTON'S PHARMACEUTICAL SCIENCES, 19th edition, (Chap. 82), Mack Publishing Company, Mack Publishing Group, Easton, Pennsylvania (1995). Thus, immunization of individuals with allergens mixed with virus like particles containing packaged DNA rich in non-methylated CG motifs should be beneficial not only before but also after the onset of allergies.

Antigen: As used herein, the term "antigen" refers to a molecule capable of being bound by an antibody or a T cell receptor (TCR) if presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a Th cell epitope and is given in adjuvant. An antigen can have one or more epitopes (B- and T- epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

A "microbial antigen" as used herein is an antigen of a microorganism and includes, but is not limited to, infectious virus, infectious bacteria, parasites and infectious fungi. Such antigens include the intact microorganism as well as natural isolates and fragments or derivatives thereof and also synthetic or recombinant compounds which are identical to or similar to natural microorganism antigens and induce an immune response specific for that microorganism. A compound is similar to a natural microorganism antigen if it induces an immune response (humoral and/or cellular) to a natural microorganism antigen. Such antigens are used routinely in the art and are well known to the skilled artisan.

Examples of infectious viruses, bacteria, and infectious fungi that are microbial antigen as used herein, are described in WO03/024481 (page 23 last paragraph to page 25 third paragraph).

The compositions and methods of the invention are also useful for treating cancer by stimulating an antigen-specific immune response against a cancer antigen. A "tumor antigen" as used herein is a compound, such as a peptide, associated with a tumor or cancer and which is capable of provoking an immune response. In particular, the compound is capable of provoking an immune response when presented in the context of an MHC molecule. Tumor antigens can be prepared from cancer cells either by preparing crude extracts of cancer cells, for example, as described in Cohen, et al., Cancer Research, 54:1055 (1994), by partially purifying the antigens, by recombinant technology or by de novo synthesis of known antigens. Tumor antigens include antigens that are antigenic portions of or are a whole tumor or cancer polypeptide. Such antigens can be isolated or prepared recombinantly or by any other means known in the art. Cancers or tumors include, but are not limited to, biliary tract cancer; brain cancer; breast cancer; cervical cancer; choriocarcinoma; colon cancer; endometrial cancer; esophageal cancer; gastric cancer; intraepithelial neoplasms; lymphomas; liver cancer; lung cancer (e.g. small cell and non-small cell); melanoma; neuroblastomas; oral cancer; ovarian cancer; pancreas cancer; prostate cancer; rectal cancer; sarcomas; skin cancer; testicular cancer; thyroid cancer; and renal cancer, as well as other carcinomas and sarcomas.

Allergens also serve as antigens in vertebrate animals. The term "allergen", as used herein, also encompasses "allergen extracts" and "allergenic epitopes." Examples of allergens include, but are not limited to: pollens (*e.g*. grass, ragweed, birch and mountain cedar); house dust and dust mites; mammalian epidermal allergens and animal danders; mold and fungus; insect bodies and insect venom; feathers; food; and drugs (*e*.*g*., penicillin).

Antigenic determinant: As used herein, the term "antigenic determinant" is meant to refer to that portion of an antigen that is specifically recognized by either B- or T-lymphocytes. B-lymphocytes responding to antigenic determinants produce antibodies, whereas T-lymphocytes respond to antigenic determinants by proliferation and establishment of effector functions critical for the mediation of cellular and/or humoral immunity.

Antigen presenting cell: As used herein, the term "antigen presenting cell" is meant to refer to a heterogenous population of leucocytes or bone marrow derived cells which possess an immunostimulatory capacity. For example, these cells are capable of generating peptides bound to MHC molecules that can be recognized by T cells. The term is synonymous with the term "accessory cell" and includes, for example, Langerhans' cells, interdigitating cells, dendritic cells, B cells and macrophages. Under some conditions, epithelial cells, endothelial cells and other, non-bone marrow derived cells may also serve as antigen presenting cells.

Association: As used herein, the term "association" as it applies to the first and second attachment sites, refers to the binding of the first and second attachment sites that is preferably by way of at least one non-peptide bond. The nature of the association may be covalent, ionic, hydrophobic, polar or any combination thereof, preferably the nature of the association is covalent, and again more preferably the association is through at least one, preferably one, non-peptide bond. As used herein, the term "association" as it applies to the first and second attachment sites, not only encompass the direct binding or association of the first and second attachment site forming the compositions of the invention but also, alternatively and preferably, the indirect association or binding of the first and second attachment site leading to the compositions of the invention, and hereby typically and preferably by using a heterobifunctional cross-linker.

Attachment Site, First: As used herein, the phrase "first attachment site" refers to an element of non-natural or natural origin, typically and preferably being comprised by the virus-like particle, to which the second attachment site typically and preferably being comprised by the antigen or antigenic determinant may associate. The first attachment site may be a protein, a polypeptide, an amino acid, a peptide, a sugar, a polynucleotide, a natural or synthetic polymer, a secondary metabolite or compound (biotin, fluorescein, retinol, digoxigenin, metal ions, phenylmethylsulfonylfluoride), or a combination thereof, or a chemically reactive group thereof. The first attachment site is located, typically and preferably on the surface, of the virus-like particle. Multiple first attachment sites are present on the surface of virus-like particle typically in a repetitive configuration. Preferably, the first attachment site is a amino acid or a chemically reactive group thereof.

Attachment Site, Second: As used herein, the phrase "second attachment site" refers to an element associated with, typically and preferably being comprised by, the antigen or antigenic determinant to which the first attachment site located on the surface of the virus-like particle may associate. The second attachment site of the antigen or antigenic determinant may be a protein, a polypeptide, a peptide, a sugar, a polynucleotide, a natural or synthetic polymer, a secondary metabolite or compound (biotin, fluorescein, retinol, digoxigenin, metal ions, phenylmethylsulfonylfluoride), or a combination thereof, or a chemically reactive group thereof. At least one second attachment site is present on the antigen or antigenic determinant. The term "antigen or antigenic determinant with at least one second attachment site" refers, therefore, to an antigen or antigenic construct comprising at least the antigen or antigenic determinant and the second attachment site. However, in particular for a second attachment site, which is of non-natural origin, i.e. not naturally occurring within the antigen or antigenic determinant, these antigen or antigenic constructs comprise an "amino acid linker".

Bound: As used herein, the term "bound" refers to binding that may be covalent, *e.g*., by chemically coupling the immunostimulatory nucleic acid of the invention to a virus-like particle, or non-covalent, *e.g*., ionic interactions, hydrophobic interactions, hydrogen bonds, etc. Covalent bonds can be, for example, ester, ether, phosphoester, amide, peptide, imide, carbon-sulfur bonds, carbon-phosphorus bonds, and the like. The term also includes the enclosement, or partial enclosement, of a substance. The term "bound" is broader than and includes terms such as "coupled," "fused," "enclosed" and "attached." Moreover, with respect to the immunostimulatory substance being bound to the virus-like particle the term "bound" also includes the enclosement, or partial enclosement, of the immunostimulatory substance. Therefore, with respect to the immunostimulatory nucleic acid being bound to the virus-like particle the term "bound" is broader than and includes terms such as "coupled," "fused," "enclosed", "packaged" and "attached." For example, the immunostimulatory nucleic acid such as the unmethylated CpG-containing oligonucleotide can be enclosed by the VLP without the existence of an actual binding, neither covalently nor non-covalently, such that the oligonucleotide is held in place by mere "packaging."

Coupled: As used herein, the term "coupled" refers to attachment by covalent bonds or by strong non-covalent interactions, typically and preferably to attachment by covalent bonds. Moreover, with respect to the coupling of the antigen to the virus-like particle the term "coupled" preferably refers to association and attachment, respectively, by at least one non-peptide bond. Any method normally used by those skilled in the art for the coupling of biologically active materials can be used in the present invention.

Fusion: As used herein, the term "fusion" refers to the combination of amino acid sequences of different origin in one polypeptide chain by in-frame combination of their coding nucleotide sequences. The term "fusion" explicitly encompasses internal fusions, *i.e.,* insertion of sequences of different origin within a polypeptide chain, in addition to fusion to one of its termini.

CpG: As used herein, the term "CpG" refers to an oligonucleotide which contains at least one unmethylated cytosine, guanine dinucleotide sequence (*e.g*. "CpG-oligonucleotides" or DNA containing a cytosine followed by guanosine and linked by a phosphate bond) and stimulates/activates, *e.g.* has a mitogenic effect on, or induces or increases cytokine expression by, a vertebrate bone marrow derived cell. For example, CpGs can be useful in activating B cells, NK cells and antigen-presenting cells, such as dendritic cells, monocytes and macrophages. The CpGs can include nucleotide analogs such as analogs containing phosphorothioester bonds and can be double-stranded or single-stranded. Generally, double-stranded molecules are more stable *in vivo,* while single-stranded molecules have increased immune activity.

Coat protein(s): As used herein, the term "coat protein(s)" refers to the protein(s) of a bacteriophage or a RNA-phage capable of being incorporated within the capsid assembly of the bacteriophage or the RNA-phage. However, when referring to the specific gene product of the coat protein gene of RNA-phages the term "CP" is used. For example, the specific gene product of the coat protein gene of RNA-phage Qβ is referred to as "Qβ CP", whereas the "coat proteins" of bacteriophage Qb comprise the "Qb CP" as well as the A1 protein. The capsid of Bacteriophage Qβ is composed mainly of the Qβ CP, with a minor content of the A1 protein. Likewise, the VLP Qβ coat protein contains mainly Qβ CP, with a minor content of A1 protein.

Epitope: As used herein, the term "epitope" refers to continuous or discontinuous portions of a polypeptide having antigenic or immunogenic activity in an animal, preferably a mammal, and most preferably in a human. An epitope is recognized by an antibody or a T cell through its T cell receptor in the context of an MHC molecule. An "immunogenic epitope," as used herein, is defined as a portion of a polypeptide that elicits an antibody response or induces a T-cell response in an animal, as determined by any method known in the art. *(See,* for example, Geysen et al., Proc. Natl. Acad Sci. USA 81:3998-4002 (1983)). The term "antigenic epitope," as used herein, is defined as a portion of a protein to which an antibody can immunospecifically bind its antigen as determined by any method well known in the art. Immunospecific binding excludes non-specific binding but does not necessarily exclude cross-reactivity with other antigens. Antigenic epitopes need not necessarily be immunogenic. Antigenic epitopes can also be T-cell epitopes, in which case they can be bound immunospecifically by a T-cell receptor within the context of an MHC molecule.

An epitope can comprise 3 amino acids in a spatial conformation which is unique to the epitope. Generally, an epitope consists of at least about 5 such amino acids, and more usually, consists of at least about 8-10 such amino acids. If the epitope is an organic molecule, it may be as small as Nitrophenyl.

Immune response: As used herein, the term "immune response" refers to a humoral immune response and/or cellular immune response leading to the activation or proliferation of B- and/or T-lymphocytes and/or antigen presenting cells. In some instances, however, the immune responses may be of low intensity and become detectable only when using at least one substance in accordance with the invention. "Immunogenic" refers to an agent used to stimulate the immune system of a living organism, so that one or more functions of the immune system are increased and directed towards the immunogenic agent. An "immunogenic polypeptide" is a polypeptide that elicits a cellular and/or humoral immune response, whether alone or linked to a carrier in the presence or absence of an adjuvant. Preferably, the antigen presenting cell may be activated.

Immunization: As used herein, the terms "immunize" or "immunization" or related terms refer to conferring the ability to mount a substantial immune response (comprising antibodies and/or cellular immunity such as effector CTL) against a target antigen or epitope. These terms do not require that complete immunity be created, but rather that an immune response be produced which is substantially greater than baseline. For example, a mammal may be considered to be immunized against a target antigen if the cellular and/or humoral immune response to the target antigen occurs following the application of methods of the invention.

Immunostimulatory nucleic acid: As used herein, the term immunostimulatory nucleic acid refers to a nucleic acid capable of inducing and/or enhancing an immune response. Immunostimulatory nucleic acids, as used herein, comprise ribonucleic acids and in particular deoxyribonucleic acids. Preferably, immunostimulatory nucleic acids contain at least one CpG motif e.g. a CG dinucleotide in which the C is unmethylated. The CG dinucleotide can be part of a palindromic sequence or can be encompassed within a non-palindromic sequence. Immunostimulatory nucleic acids not containing CpG motifs as described above encompass, by way of example, nucleic acids lacking CpG dinucleotides, as well as nucleic acids containing CG motifs with a methylated CG dinucleotide. The term "immunostimulatory nucleic acid" as used herein should also refer to nucleic acids that contain modified bases such as 4-bromo-cytosine.

Immunostimulatory substance: As used herein, the term "immunostimulatory substance" refers to a substance capable of inducing and/or enhancing an immune response. Immunostimulatory substances, as used herein, include, but are not limited to, toll-like receptor activing substances and substances inducing cytokine secretion. Toll-like receptor activating substances include, but are not limited to, immunostimulatory nucleic acids, peptideoglycans, lipopolysaccharides, lipoteichonic acids, imidazoquinoline compounds, flagellins, lipoproteins, and immunostimulatory organic substances such as taxol.

Mixed: As used herein, the term "mixed" refers to the combination of two or more substances, ingredients, or elements that are added together, are not chemically combined with each other and are capable of being separated.

Oligonucleotide: As used herein, the terms "oligonucleotide" or "oligomer" refer to a nucleic acid sequence comprising 2 or more nucleotides, generally at least about 6 nucleotides to about 100,000 nucleotides, preferably about 6 to about 2000 nucleotides, and more preferably about 6 to about 300 nucleotides, even more preferably about 20 to about 300 nucleotides, and even more preferably about 20 to about 100 nucleotides. The terms "oligonucleotide" or "oligomer" also refer to a nucleic acid sequence comprising more than 100 to about 2000 nucleotides, preferably more than 100 to about 1000 nucleotides, and more preferably more than 100 to about 500 nucleotides. "Oligonucleotide" also generally refers to any polyribonucleotide or polydeoxribonucleotide, which may be unmodified RNA or DNA or modified RNA or DNA. The modification may comprise the backbone or nucleotide analogues. "Oligonucleotide" includes, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "oligonucleotide" refers to triple-stranded regions comprising RNA or DNA or both RNA and DNA. Further, an oligonucleotide can be synthetic, genomic or recombinant, *e.g*., λ-DNA, cosmid DNA, artificial bacterial chromosome, yeast artificial chromosome and filamentous phage such as M13.

The term "oligonucleotide" also includes DNAs or RNAs containing one or more modified bases and DNAs or RNAs with backbones modified for stability or for other reasons. For example, suitable nucleotide modifications/analogs include peptide nucleic acid, inosin, tritylated bases, phosphorothioates, alkylphosphorothioates, 5-nitroindole deoxyribofuranosyl, 5-methyldeoxycytosine and 5,6-dihydro-5,6-dihydroxydeoxythymidine. A variety of modifications have been made to DNA and RNA; thus, "oligonucleotide" embraces chemically, enzymatically or metabolically modified forms of polynucleotides as typically found in nature, as well as the chemical forms of DNA and RNA characteristic of viruses and cells. Other nucleotide analogs/modifications will be evident to those skilled in the art.

Packaged: The term "packaged" as used herein refers to the state of an immunostimulatory substance, in particular an immunostimulatory nucleic acid in relation to the VLP. The term "packaged" as used herein includes binding that may be covalent, *e.g*., by chemically coupling, or non-covalent, *e.g*., ionic interactions, hydrophobic interactions, hydrogen bonds, etc. Covalent bonds can be, for example, ester, ether, phosphoester, amide, peptide, imide, carbon-sulfur bonds, carbon-phosphorus bonds, and the like. The term "packaged" includes terms such as "coupled" and "attached", and in particular, and preferably, the term "packaged" also includes the enclosement, or partial enclosement, of a substance. For example, the immunostimulatory substance such as the unmethylated CpG-containing oligonucleotide can be enclosed by the VLP without the existence of an actual binding, neither covalently nor non-covalently. Therefore, in the preferred meaning, the term "packaged", and hereby in particular, if immunostimulatory nucleic acids are the immunostimulatory substances, the term "packaged" indicates that the nucleic acid in a packaged state is not accessible to DNAse or RNAse hydrolysis. In preferred embodiments, the immunostimulatory nucleic acid is packaged inside the VLP capsids, most preferably in a non-covalent manner.

PCR product: As used herein, the term "PCR product" refers to amplified copies of target DNA sequences that act as starting material for a PCR. Target sequences can include, for example, double-stranded DNA. The source of DNA for a PCR can be complementary DNA, also referred, to as "cDNA", which can be the conversion product of mRNA using reverse transcriptase. The source of DNA for a PCR can be total genomic DNA extracted from cells. The source of cells from which DNA can be extracted for a PCR includes, but is not limited to, blood samples; human, animal, or plant tissues; fungi; and bacteria. DNA starting material for a PCR can be unpurified, partially purified, or highly purified. The source of DNA for a PCR can be from cloned inserts in vectors, which includes, but is not limited to, plasmid vectors and bacteriophage vectors. The term "PCR product" is interchangeable with the term "polymerase chain reaction product".

The compositions of the invention can be combined, optionally, with a pharmaceutically-acceptable carrier. The term "pharmaceutically-acceptable carrier" as used herein means one or more compatible solid or liquid fillers, diluents or encapsulating substances which are suitable for administration into a human or other animal. The term "carrier" denotes an organic or inorganic ingredient, natural or synthetic, with which the active ingredient is combined to facilitate the application.

Polypeptide: As used herein, the term "polypeptide" refers to a molecule composed of monomers (amino acids) linearly linked by amide bonds (also known as peptide bonds). It indicates a molecular chain of amino acids and does not refer to a specific length of the product. Thus, peptides, oligopeptides and proteins are included within the definition of polypeptide. This term is also intended to refer to post-expression modifications of the polypeptide, for example, glycosolations, acetylations, phosphorylations, and the like. A recombinant or derived polypeptide is not necessarily translated from a designated nucleic acid sequence. It may also be generated in any manner, including chemical synthesis.

A substance which "enhances" an immune response refers to a substance in which an immune response is observed that is greater or intensified or deviated in any way with the addition of the substance when compared to the same immune response measured without the addition of the substance. For example, the lytic activity of cytotoxic T cells can be measured, *e.g*. using a ⁵¹Cr release assay, with and without the substance. The amount of the substance at which the CTL lytic activity is enhanced as compared to the CTL lytic activity without the substance is said to be an amount sufficient to enhance the immune response of the animal to the antigen. In a preferred embodiment, the immune response in enhanced by a factor of at least about 2, more preferably by a factor of about 3 or more. The amount or type of cytokines secreted may also be altered. Alternatively, the amount of antibodies induced or their subclasses may be altered.

Effective Amount: As used herein, the term "effective amount" refers to an amount necessary or sufficient to realize a desired biologic effect. An effective amount of the composition would be the amount that achieves this selected result, and such an amount could be determined as a matter of routine by a person skilled in the art. For example, an effective amount for treating an immune system deficiency could be that amount necessary to cause activation of the immune system, resulting in the development of an antigen specific immune response upon exposure to antigen. The term is also synonymous with "sufficient amount."

The effective amount for any particular application can vary depending on such factors as the disease or condition being treated, the particular composition being administered, the size of the subject, and/or the severity of the disease or condition. One of ordinary skill in the art can empirically determine the effective amount of a particular composition of the present invention without necessitating undue experimentation.

Toll-like receptor (TLR) ligand: As used herein, the term "Toll-like receptor ligand" or "TLR ligand" refers to any ligand which is capable of activating at least one of the TLRs (see e.g. Beutler, B. 2002, Curr. Opin. Hematol., 9, 2-10, Schwarz et al., 2003, Eur.J.Immunol., 33, 1465-1470). A TLR ligand of the invention activates without limitation at least one toll-like receptor I (TLR1), TLR2, TLR3, TLR4, TLR5, TLR6, TLR7, TLR8, TLR9, TLR10, or TLR11. For example, peptidoglycan (PGN) or lipoteichoic acid (LTA) typically and preferably activates TLR2 (Aliprantis et al., Science (1999), 285:736-9; Underhill, et al., Nature, (1999), 401:811-5); double-stranded RNA, e.g. poly (I:C), typically and preferably activates TLR3 (Alexopoulou et al., Nature (2001), 413:732-8); lipopolysachride (LPS) typically and preferably activates TLR4 (Poltorak, el at., Science (1998), 282:2085-8); flagellin typically and preferably activates TLR5 (Hayashi et al. Nature (2001), 410:1099-103); single stranded RNA, for example bacterial RNA, and certain synthetic substances such as imidazoquinolines, typically and preferably activate TLR7 and TLR8 (Diebold S. et al. Science 303:1529; Heil, FH. et al. Science 303:1526); bacterial DNA, in particular DNA containing CpG motifs typically and preferably activates TLR9 (Schnare et al. Curr. Biol. (2000), 10:1139-42; Hemmi H et al. Nature (2000), 408: 740-5). A summary of TLR ligands is given in the table of Abreu's review paper (Abreu M.T. and Arditi M. J., Pediatrics (2004), 421-9) and a reference for TLR 11 and its ligand is described in Zhang et al., Science, (2004), 303:1522-6. By referring to these papers in conjunction with general knowledge of a skilled person in the art, it is within a routine practice to test whether a molecule is a TLR ligand in accordance with the present invention, and whether a TLR ligand activates at least one of the TLR. A typical and preferred example for such testing is as follows: 3x10⁶ HEK293 cells are electroporated at 200 volt and 960 µF with 1 µg of TLR expression plasmid and 20 ng NF-kB luciferase reporter-plasmid. The overall amount of plasmid DNA is held constant at 15 µg per electroporation by addition of the appropriate empty expression vector. Cells are seeded at 10⁵ cells per well and after overnight culture stimulated with the ligand to be tested for a further 7 to 10 hours. Typical examples of concentration ranges for known TLR ligands are 25 µg/ ml RNA40-42 complexed to DOTAP (facilitating the internalization of RNA inside the cell), 1 µM CpG-ODN 2006, 10 µMR-848, 50 µg/ml poly(I:C) or 1 µg/ml Pam3Cys (Heil, FH. et al. Science 303:1526). Stimulated cells are lysed using reporter lysis buffer (Promega, Mannheim, Germany) and lysate is assayed for luciferase activity using a luminometer , typically and preferably the Berthold luminometer (Wildbad, Germany), according to the manufacturer's instruction. It is within the knowledge of the skilled person in the art to accordingly adapt the aforementioned experiment for the testing of any ligand.

A ligand is, then, considered to activate a TLR in accordance with this invention, when the induced luciferase activity is statistically significantly higher than a threshold value determined from the the activitiy of the negative control (identical experiment and identical experimental conditions without the addition of the ligand to be tested). A threshold value within this context is defined by the mean of the luciferase activities of the negative control in six independent experiments plus three times the standard deviation of the luciferase activities from the six experiments. A ligand is, then typically and preferably, considered to "statistically significantly" activate a TLR when the luciferase activity of the ligand is higher than the threshold value determined as indicated above. Preferably, a ligand is considered to "statistically significantly" activate a TLR when the luciferase activity of the ligand is at least two times higher, preferably three times higher, even more preferably five times higher than the threshold value determined as indicated above.

In case an immunostimulatory nucleic acid activates a TLR, the further TLR ligand (d) as described activates a TLR that is different from the TLR activated by the immunostimulatory nucleic acid. If, for example, the immunostimulatory nucleic acid is CpG, a ligand for TLR9, the TLR ligand (d) of the composition as described and thus the second TLR ligand activates a second TLR which is any TLR other than TLR9, and activates for example, TLR1, 2, 3, 4, 5, 6, 7, 8, 10, or 11. On the other hand, if for example the immunostimulatory nucleic acid is poly (I:C), a ligand for TLR3, the TLR ligand (d) of the composition as described, and thus the second TLR ligand activates a second TLR which is any TLR other than TLR 3, and activates for example TLR1, 2, 4, 5, 6, 7, 8, 9, 10, or 11.

Toll-like receptor 4 (TLR4) ligands: TLR4 ligands are able to signal into a cell in a TLR4-dependent fashion. A typical and preferred example is LPS and derivatives thereof, gp96, heat-shock proteins and defensins. Preferred TLR 4 ligands are LPS and its derivatives such as detoxified versions of LPS which lack for example side chains of the lipid A tail (Persing et al., (2002), Trends Microbiol., 10 (10 Suppl), 32-37), such as MPL, Monophosphoryl lipid A and derivatives thereof (Johnson et al., (1999), J Med Chem., 42(22), 4640-4649) or chemically altered and synthethic analoga of LPS (Fernandes et al, (1997), 34 (8-9) 569-576; Przetak et al, (2003), 21, 961-970). All the cited references are included herein in its entirety. Preferred LPS derivatives of the present inventions, such as detoxified versions of LPS, chemically altered or synthethic analoga of LPS are subjected to a pyrogenicity test in rabbits as known by the skilled person in the art. Typically and preferably, a preferred LPS derivative shows no, or no significant pyrogenicity test in rabbits

Self antigen: As used herein, the tem "self antigen" refers to proteins encoded by the host's genome or DNA and products generated by proteins or RNA encoded by the host's genome or DNA are defined as self. Preferably, the tem "self antigen", as used herein, refers to proteins encoded by the human genome or DNA and products generated by proteins or RNA encoded by the human genome or DNA are defined as self. The inventive compositions, pharmaceutical compositions and vaccines comprising self antigens are in particular capable of breaking tolerance against a self antigen when applied to the host. In this context, "breaking tolerance against a self antigen" shall refer to enhancing an immune response, as defined herein, and preferably enhancing a B or a T cell response, specific for the self antigen when applying the inventive compositions, pharmaceutical compositions and vaccines comprising the self antigen to the host. In addition, proteins that result from a combination of two or several self-molecules or that represent a fraction of a self-molecule and proteins that have a high homology two self-molecules as defined above (>95%, preferably >97%, more preferably >99%) may also be considered self. In a further preferred embodiment of the present invention, the antigen is a self antigen. Very preferred embodiments of self-antigens useful for the present invention are described WO 02/056905.

Treatment: As used herein, the terms "treatment", "treat", "treated" or "treating" refer to prophylaxis and/or therapy. When used with respect to an infectious disease, for example, the term refers to a prophylactic treatment which increases the resistance of a subject to infection with a pathogen or, in other words, decreases the likelihood that the subject will become infected with the pathogen or will show signs of illness attributable to the infection, as well as a treatment after the subject has become infected in order to fight the infection, *e.g*., reduce or eliminate the infection or prevent it from becoming worse.

Vaccine: As used herein, the term "vaccine" refers to a formulation which contains the composition of the present invention and which is in a form that is capable of being administered to an animal. Typically, the vaccine comprises a conventional saline or buffered aqueous solution medium in which the composition of the present invention is suspended or dissolved. In this form, the composition of the present invention can be used conveniently to prevent, ameliorate, or otherwise treat a condition. Upon introduction into a host, the vaccine is able to provoke an immune response including, but not limited to, the production of antibodies and/or cytokines and/or the activation of cytotoxic T cells, antigen presenting cells, helper T cells, dendritic cells and/or other cellular responses.

Optionally, the vaccine additionally includes an adjuvant which can be present in either a minor or major proportion relative to the compound of the present invention. The term "adjuvant" as used herein refers to non-specific stimulators of the immune response or substances that allow generation of a depot in the host which when combined with the vaccine of the present invention provide for an even more enhanced immune response. A variety of adjuvants can be used. Examples include incomplete Freund's adjuvant, aluminum hydroxide and modified muramyldipeptide.

Virus-like particle: As used herein, the term "virus-like particle" (VLP) refers to a structure resembling a virus but which has not been demonstrated to be pathogenic. Typically, a virus-like particle in accordance with the invention does not carry genetic information encoding for the proteins of the virus-like particle. In general, virus-like particles lack the viral genome and, therefore, are noninfectious. Also, virus-like particles can often be produced in large quantities by heterologous expression and can be easily purified. Some virus-like particles may contain nucleic acid distinct from their genome. Typically, a virus-like particle in accordance with the invention is non replicative and noninfectious since it lacks all or part of the viral genome, in particular the replicative and infectious components of the viral genome. A virus-like particle in accordance with the invention may contain nucleic acid distinct from their genome. A typical and preferred embodiment of a virus-like particle in accordance with the present invention is a viral capsid such as the viral capsid of the corresponding virus, bacteriophage, or RNA-phage. The terms "viral capsid" or "capsid", as interchangeably used herein, refer to a macromolecular assembly composed of viral protein subunits. Typically and preferably, the viral protein subunits assemble into a viral capsid and capsid, respectively, having a structure with an inherent repetitive organization, wherein said structure is, typically, spherical or tubular. For example, the capsids of RNA-phages or HBcAg's have a spherical form of icosahedral symmetry. The term "capsid-like structure" as used herein, refers to a macromolecular assembly composed of viral protein subunits ressembling the capsid morphology in the above defined sense but deviating from the typical symmetrical assembly while maintaining a sufficient degree of order and repetitiveness.

VLP of RNA phage coat protein: The capsid structure formed from the self-assembly of 180 subunits of RNA phage coat protein and optionally containing host RNA is referred to as a "VLP of RNA phage coat protein". A specific example is the VLP of Qβ coat protein. In this particular case, the VLP of Qβ coat protein may either be assembled exclusively from Qβ CP subunits (SEQ ID NO: 1) generated by expression of a Qβ CP gene containing, for example, a TAA stop codon precluding any expression of the longer A1 protein through suppression, see Kozlovska, T.M., et al., Intervirology 39: 9-15 (1996)), or additionally contain A1 protein subunits (SEQ ID NO: 2) in the capsid assembly. The readthrough process has a low efficiency and is leading to an only very low amount A1 protein in the VLPs. An extensive number of examples have been performed with different combinations of ISS packaged and antigen coupled. No differences in the coupling efficiency and the packaging have been observed when VLPs of Qβ coat protein assembled exclusively from Qβ CP subunits or VLPs of Qβ coat protein containing additionally A1 protein subunits in the capsids were used. Furthermore, no difference of the immune response between these Qβ VLP preparations was observed. Therefore, for the sake of clarity the term "Qβ VLP" is used throughout the description of the examples either for VLPs of Qβ coat protein assembled exclusively from Qβ CP subunits or VLPs of Qβ coat protein containing additionally A1 protein subunits in the capsids.

The term "virus particle" as used herein refers to the morphological form of a virus. In some virus types it comprises a genome surrounded by a protein capsid; others have additional structures (*e.g*., envelopes, tails, etc.).

Non-enveloped viral particles are made up of a proteinaceous capsid that surrounds and protects the viral genome. Enveloped viruses also have a capsid structure surrounding the genetic material of the virus but, in addition, have a lipid bilayer envelope that surrounds the capsid.

In a preferred embodiment of the invention, the VLP's are free of a lipoprotein envelope or a lipoprotein-containing envelope. In a further preferred embodiment, the VLP's are free of an envelope altogether.

One, a, or an: When the terms "one," "a," or "an" are used in this disclosure, they mean "at least one" or "one or more," unless otherwise indicated.

As will be clear to those skilled in the art, certain embodiments of the invention involve the use of recombinant nucleic acid technologies such as cloning, polymerase chain reaction, the purification of DNA and RNA, the expression of recombinant proteins in prokaryotic and eukaryotic cells, etc. Such methodologies are well known to those skilled in the art and can be conveniently found in published laboratory methods manuals (*e.g*., Sambrook, J. et al., eds., MOLECULAR CLONING, A LABORATORY MANUAL, 2nd, edition, Cold Spring Harbor Laboratory Press, Cold Spring Harbor, N.Y. (1989); Ausubel, F. et al., eds., CURRENT PROTOCOLS IN MOLECULAR BIOLOGY, John H. Wiley & Sons, Inc. (1997)). Fundamental laboratory techniques for working with tissue culture cell lines (Celis, J., ed., CELL BIOLOGY, Academic Press, 2nd edition, (1998)) and antibody-based technologies (Harlow, E. and Lane, D., "Antibodies: A Laboratory Manual," Cold Spring Harbor Laboratory, Cold Spring Harbor, N.Y. (1988); Deutscher, M.P., "Guide to Protein Purifcation," Meth. Enzymol. 128, Academic Press San Diego (1990); Scopes, R.K., "Protein Purification Principles and Practice," 3rd ed., Springer-Verlag, New York (1994)) are also adequately described in the literature.

### 2. Compositions and Methods for Enhancing an Immune Response

Disclosed herein are compositions and methods for enhancing an immune response against one or more antigens in an animal. The disclosed compositions comprise, or alternatively consist of, (a) a virus-like particle, (b) an immunostimulatory nucleic acid, preferably an unmethylated CpG-containing oligonucleotide where the nucleic acid or oligonucleotide is coupled to, fused to, or otherwise attached to or enclosed by, *i.e*., bound to, and preferably packaged with the virus-like particle, (c) at least one antigen coupled or fused to the VLP or an antigen mixed with the VLP, and (d) at least one ligand for a TLR. Preferably, the TLR ligand (d) is mixed with the VLP (a). Furthermore, the disclosure conveniently enables the practitioner to construct such a composition for various treatment and/or prevention purposes, which include the prevention and/or treatment of infectious diseases, as well as chronic infectious diseases, the prevention and/or treatment of cancers, and the prevention and/or treatment of allergies or allergy-related diseases such as asthma, for example.

Virus-like particles in the context of the present application refer to VLPs that are desribed in detail in WO 03/024481 on page 39 to 59. Examples of VLPs include, but are not limited to, the capsid proteins of Hepatitis B virus, RNA phages, Ty, fr-phage, GA-phage, AP 205-phage and, in particular, Qβ-phage. The VLP can comprise, or alternatively essentially consist of, or alternatively consist of recombinant polypeptides, or fragments thereof. The virus-like particle may comprise, consist essentially of or alternatively consist of recombinant proteins, or fragments thereof, of a RNA-phage. Preferably, the RNA-phage is selected from the group consisting of a) bacteriophage Qβ; b) bacteriophage R17; c) bacteriophage fr; d) bacteriophage GA; e) bacteriophage SP; f) bacteriophage MS2; g) bacteriophage M11; h) bacteriophage MX1; i) bacteriophage NL95; k) bacteriophage f2; 1) bacteriophage PP7; and m) bacteriophage AP205. The recombinant proteins comprise, consist essentially of or alternatively consist of coat proteins of RNA phages.

Specific preferred examples of bacteriophage coat proteins which can be used to prepare compositions of the invention are described in detail in WO 03/024481 (page 41 last paragraph to page 49 second paragraph), , and which include the coat proteins of RNA bacteriophages such as bacteriophage Qβ (PIR Database, Accession No. VCHPQβ referring to Qβ CP and Accession No. AAA16663 referring to Qβ A1 protein), bacteriophage R17 (PIR Accession No. VCBPR7), bacteriophage fr (PIR Accession No. VCBPFR), bacteriophage GA (GenBank Accession No. NP-040754), bacteriophage SP (GenBank Accession No. CAA30374 referring to SP CP and Accession No. NP 695026 referring to SP *A1 protein), bacteriophage MS2 (PIR Accession No. VCBPM2), bacteriophage M11 (GenBank Accession No. AAC06250), bacteriophage MX1 (GenBank Accession No. AAC14699), bacteriophage NL95 (GenBank Accession No. AAC 14704), bacteriophage f2 (GenBank Accession No. P03611), bacteriophage PP7 (SEQ ID NO: 3), bacteriophage AP205 (SEQ ID NO: 32 or 33).

Four lysine residues are exposed on the surface of the capsid of Qβ coat protein. Qβ mutants, for which exposed lysine residues are replaced by arginines, can also be used for the present invention. The following Qβ coat protein mutants and mutant Qβ VLP's can, thus, be used in the practice of the invention: "Qβ-240" (Lys13-Arg; SEQ ID NO:4), "Qβ-243" (Asn 10-Lys; SEQ ID NO:5), "Qβ-250" (Lys 2-Arg, Lys13-Arg; SEQ ID NO:6), "Qβ-251" (SEQ ID NO:7) and "Qβ-259" (Lys 2-Arg, Lys16-Arg; SEQ ID NO:8). Thus, in further preferred embodiment of the present invention, the virus-like particle comprises, consists essentially of or alternatively consists of recombinant proteins of mutant Qβ coat proteins, which comprise proteins having an amino acid sequence selected from the group of a) the amino acid sequence of SEQ ID NO:4; b) the amino acid sequence of SEQ ID NO:5; c) the amino acid sequence of SEQ ID NO:6; d) the amino acid sequence of SEQ ID NO:7; and e) the amino acid sequence of SEQ ID NO:8. The construction, expression and purification of the above indicated Qβ coat proteins, mutant Qβ coat protein VLP's and capsids, respectively, are described in WO 02/056905.

Further described are composition comprising proteins which comprise, or alternatively consist essentially of, or alternatively consist of, amino acid sequences which are at least 80%, 85%, 90%, 95%, 97%, or 99% identical to the above described. Fragments of VLPs which retain the ability to induce an immune response can comprise, or alternatively consist of, polypeptides which are about 15, 20, 25, 30, 35, 40, 45, 50, 55, 60, 65, 70, 75, 80, 85, 90, 95, 100, 110, 120, 130, 140, 150, 160, 170, 180, 190, 200, 250, 300, 350, 400, 450 or 500 amino acids in length, but will obviously depend on the length of the sequence of the subunit composing the VLP. Examples of such fragments include fragments of proteins discussed herein which are suitable for the preparation of the immune response enhancing composition.

As previously stated, the invention includes virus-like particles or recombinant forms thereof. Skilled artisans have the knowledge to produce such particles and mix antigens thereto. The specification also provides for the production of Hepatitis B virus-like particles as virus-like particles (Example 1).

Particles used in compositions disclosed herein are composed of a Hepatitis B capsid (core) protein (HBcAg) or a fragment of a HBcAg, or HBcAg which has been modified to either eliminate or reduce the number of free cysteine residues. Specific preferred examples of HBcAg proteins, such as for example the HBcAg of SEQ ID NO: 9 or variants thereof, which can be used to prepare compositions of the invention are described in detail in WO 03/024481 (page 52 fourth paragraph to page 58 last paragraph) .The preparation of Hepatitis B virus-like particles ,is disclosed, for example, in WO 00/32227, and hereby in particular in Examples 17 to 19 and 21 to 24, as well as in WO 01/85208, and hereby in particular in Examples 17 to 19, 21 to 24, 31 and 41, and in WO 02/056905. For the latter application, it is in particular referred to Example 23, 24, 31 and 51.

A number of naturally occurring HBcAg variants have been identified (e.g. Yuan et al., (J. Virol 73:10122-10128 (1999)). Further HBcAg variants are disclosed in WO 03/024481 (page 54 third paragraph to page 55 first paragraph)

HbcAgs can be derived from any organism so long as they are able to enclose or to be coupled or otherwise attached to an unmethylated CpG-containing oligonucleotide and induce an immune response.

A lysine residue may be introduced into a HBcAg polypeptide, to mediate the binding of the antigen or antigenic determinant to the VLP of HBcAg. Compositions disclosed herein are prepared using a HBcAg comprising, or alternatively consisting of, amino acids 1-144, or 1-149, or 1-185 of SEQ ID NO:10, which is modified so that the amino acids corresponding to positions 79 and 80 are replaced with a peptide having the amino acid sequence of Gly-Gly-Lys-Gly-Gly (SEQ ID NO:34), resulting in the HBcAg variant having the amino acid sequence of SEQ ID NO: 96. In further aspects the cysteine residues at positions 48 and 107 of SEQ ID NO: 10 are mutated to serine (SEQ ID NO: 36). Further disclosed are compositions comprising the corresponding polypeptides having amino acid sequences shown in WO 03/024481 (page 54 third paragraph to page 55 first paragraph), which also have above noted amino acid alterations. Further disclosed are additional HBcAg variants which are capable of associating to form a capsid or VLP and have the above noted amino acid alterations. Thus, also disclosed are compositions comprising HBcAg polypeptides which comprise, or alternatively consist of, amino acid sequences which are at least 80%, 85%, 90%, 95%, 97% or 99% identical to any of the wild-type amino acid sequences, and forms of these proteins which have been processed, where appropriate, to remove the N-terminal leader sequence and modified with above noted alterations.

In one aspect a virus-like particle, to which an unmethylated CpG-containing oligonucleotide is bound, is coupled to or mixed with antigen/immunogen against which an enhanced immune response is desired. In some instances, a single antigen will be coupled to or mixed with the so modified virus-like particle. In other instances, the so modified VLPs will be coupled to or mixed with several antigens or even complex antigen mixtures. The antigens can be produced recombinantly or be extracted from natural sources, which include but are not limited to pollen, dust, fungi, insects, food, mammalian epidermals, feathers, bees, tumors, pathogens and feathers.

The substance that is added to the composition comprising a VLP containing at least one immunostimulatory nucleic acid, preferably at least one unmethylated CpG-containing oligonucleotide, and antigen, either coupled/fused to the VLP or mixed with the VLP, and at least one TLR ligand, is able to trigger activation of a TLR, typically and preferably a second TLR which is not activated by the immunostimulatory nucleic acid of the invention.

Further disclosed is a composition for enhancing an immune response in an animal comprising (a) a virus-like particle (VLP), (b) an immunostimulatory nucleic acid, preferably an unmethylated CpG-containing oligonucleotide, where the nucleic acid or oligonucleotide is coupled to, fused to, or otherwise attached to or enclosed by, *i.e*., bound to, and preferably packaged with the virus-like particle, (c) at least one antigen coupled or fused to the VLP or an antigen mixed with the VLP, and (d) at least one TLR ligand. Preferably, the TLR ligand (d) is mixed with the VLP (a) . Preferably, the immunostimulatory nucleic acid (b) activates a TLR that is different than the TLR activated by the ligand (d).

TLRs are well described pattern recognition molecules that are key for self/non-self discrimination by the immune system. Ten human toll-like receptors are known uptodate. They are activated by a variety of ligands. TLR2 is activated by peptidoglycans, lipoproteins, lipopolysacchrides, lipoteichonic acid and Zymosan, and macrophage-activating lipopeptide MALP-2; TLR3 is activated by double-stranded RNA such as poly (I:C); TLR4 is activated by lipopolysaccharide, lipoteichoic acids and taxol and heat-shock proteins such as heat shock protein HSP-60, Gp96 and defensins; TLR5 is activated by bacterial flagella, especially the flagellin protein; TLR6 is activated by peptidoglycans, TLR7 is activated by imiquimoid and imidazoquinoline compounds, such as R-848, loxoribine and bropirimine and TLR9 is activated by bacterial DNA, in particular CpG-oligonucleotides. Ligands for TLR1, TLR8, TLR10, and TLR 11 are not known so far. However, recent reports indicate that same receptors can react with different ligands and that further receptors are present. The above list of ligands is not exhaustive and further ligands are within the knowledge of the person skilled in the art. In general, triggering of TLRs leads to the activation of antigen presenting cells (APC). Thus, triggering of TLRs may enhance T cell responses by activation of APCs. In the present invention, we made the suprising finding that stimulation of different TLRs may lead to a synergistic response. Specifically, stimulation of TLR9 by CpGs packaged into VLPs may synergize with stimulation of TLR4 by LPS or other TLR4 ligands. Thus, the TLR stimulated additionally to TLR9 by CpGs is TLR4. Various ligands are known for TLR4. Those include LPS, which are the natural ligand of TLR4. In addition, detoxified versions of LPS, which lack e.g. side chains of the lipid A tail, are also potent activators of TLR4. Monosphoryl lipid A and derivatives thereof are known in the art. A preferred derivative is 3 de-o-acylated monophosphoryl lipid A, and is known from British Patent No. 2220211. Despite their ability to stimulate TLR4, these non-natural ligands are relatively non-toxic and therefore preferred substances for stimulation TLR4 in vaccine formulations (Curr Drug Targets Infect Disord. 2001 Nov;1(3):273-86.). Recently, the family of TLR ligands has expanded to include heat shock proteins (J Biol Chem. 2002 Apr 26;277(17):15107-12.) and defensins (Science. 2002 Nov 1;298(5595):1025-9.). Thus, defensins and heat-shock proteins are also described.

The immunostimulatory nucleic acid may be selected from the group consisting of (a) ribonucleic acids; (b) deoxyribonucleic acids, (c) chimeric nucleic acids; and (d) any mixtures of at least one nucleic acid of (a), (b) and/or (c). The immunostimulatory nucleic acid may be poly-(I:C). The immunostimulatory nucleic acid may be selected from the group consisting of (a) unmethylated CpG-containing oligonucleotides; and (b) oligonucleotides free of unmethylated CpG motifs, preferably the immunostimulatory nucleic acid of the invention is unmethylated CpG-containing oligonucleotide.

Two classes of nucleic acids, namely 1) bacterial DNA that contains immunostimulatory sequences, in particular unmethylated CpG dinucleotides within specific flanking bases (referred to as CpG motifs) and 2) double-stranded RNA synthesized by various types of viruses represent important members of the microbial components that enhance immune responses. Synthetic double stranded (ds) RNA such as polyinosinic-polycytidylic acid (poly I:C) are capable of inducing dendritic cells to produce proinflammatory cytokines and to express high levels of costimulatory molecules.

A series of studies by Tokunaga and Yamamoto et al. has shown that bacterial DNA or synthetic oligodeoxynucleotides induce human PBMC and mouse spleen cells to produce type I interferon (IFN) (reviewed in Yamamoto et al., Springer Semin Immunopathol. 22:11-19). Poly (I:C) was originally synthesized as a potent inducer of type I IFN but also induces other cytokines such as IL-12.

Ribonucleic acids encompass polyinosinic-polycytidylic acid double-stranded RNA (poly I:C). Ribonucleic acids and modifications thereof as well as methods for their production have been described by Levy, H.B (Methods Enzymol. 1981, 78:242-251), DeClercq, E (Methods Enzymol. 1981, 78: 227-236) and Torrence, P.F. (Methods Enzymol 1981;78:326-331) and references therein. Further ribonucleic acids comprise polynucleotides of inosinic acid and cytidiylic acid such poly (IC) of which two strands forms double stranded RNA. Ribonucleic acids can be isolated from organisms. Ribonucleic acids also encompass further synthetic ribonucleic acids, in particular synthetic poly (I:C) oligonucleotides that have been rendered nuclease resistant by modification of the phosphodiester backbone, in particular by phosphorothioate modifications. The ribose backbone of poly (I:C) may also be replaced by a deoxyribose. Those skilled in the art know procedures how to synthesize synthetic oligonucleotides.

In general, the unmethylated CpG-containing oligonucleotide comprises the sequence:

5' X₁X₂CGX₃X₄ 3'

wherein X₁, X₂, X₃ and X₄ are any nucleotide. In addition, the oligonucleotide can comprise about 6 to about 100,000 nucleotides, preferably about 6 to about 2000 nucleotides, more preferably about 20 to about 2000 nucleotides, and even more preferably comprises about 20 to about 300 nucleotides. In addition, the oligonucleotide can comprise more than 100 to about 2000 nucleotides, preferably more than 100 to about 1000 nucleotides, and more preferably more than 100 to about 500 nucleotides.

The CpG-containing oligonucleotide may contains one or more phosphothioester modifications of the phosphate backbone. For example, a CpG-containing oligonucleotide having one or more phosphate backbone modifications or having all of the phosphate backbone modified and a CpG-containing oligonucleotide wherein one, some or all of the nucleotide phosphate backbone modifications are phosphorothioate modifications are described.

The CpG-containing oligonucleotide can also be recombinant, genomic, synthetic, cDNA, plasmid-derived and single or double stranded. For use in the instant invention, the nucleic acids can be synthesized de novo using any of a number of procedures well known in the art. For example, the b-cyanoethyl phosphoramidite method (Beaucage, S. L., and Caruthers, M. H., Tet. Let. 22:1859 (1981); nucleoside H-phosphonate method (Garegg et al., Tet. Let. 27:4051-4054 (1986); Froehler et al., Nucl. Acid Res. 14:5399-5407 (1986); Garegg et al., Tet. Let. 27:4055-4058 (1986), Gaffney et al., Tet. Let. 29:2619-2622 (1988)). These chemistries can be performed by a variety of automated oligonucleotide synthesizers available in the market. Alternatively, CpGs can be produced on a large scale in plasmids, (see Sambrook, T., et al., "Molecular Cloning: A Laboratory Manual," Cold Spring Harbor laboratory Press, New York, 1989) which after being administered to a subject are degraded into oligonucleotides. Oligonucleotides can be prepared from existing nucleic acid sequences (e.g., genomic or cDNA) using known techniques, such as those employing restriction enzymes, exonucleases or endonucleases.

The immunostimulatory nucleic acids as well as the unmethylated CpG-containing oligonucleotide can be bound to the VLP by any way known is the art provided the composition enhances an immune response in an animal. For example, the oligonucleotide can be bound either covalently or non-covalently. In addition, the VLP can enclose, fully or partially, the immunostimulatory nucleic acids as well as the unmethylated CpG-containing oligonucleotide. The immunostimulatory nucleic acid as well as the unmethylated CpG-containing oligonucleotide can be bound to a VLP site such as an oligonucleotide binding site (either naturally or non-naturally occurring), a DNA binding site or a RNA binding site. In another embodiment, the VLP site comprises an arginine-rich repeat or a lysine-rich repeat. Methods of packaging an immunostimulatory nucleic acid of the invention to a VLP of the invention, e.g. to HBcAg, RNA-phage Qβ or AP205 is known in the art and has been described in WO 03/024481 (in particular in Examples 11 to 17)

The compositions of the invention may be specifically used to activate dendritic cells for the purpose of enhancing a specific immune response against antigens. The dendritic cells can be enhanced using ex vivo or in vivo techniques. The ex vivo procedure can be used on autologous or heterologous cells, but is preferably used on autologous cells. The dendritic cells may be isolated from peripheral blood or bone marrow, but can be isolated from any source of dendritic cells. Ex vivo manipulation of dendritic cells for the purposes of cancer immunotherapy have been described in several references in the art, including Engleman, E. G., Cytotechnology 25:1 (1997); Van Schooten, W., et al., Molecular Medicine Today, June, 255 (1997); Steinman, R. M., Experimental Hematology 24:849 (1996); and Gluckman, J. C., Cytokines, Cellular and Molecular Therapy 3:187 (1997).

The dendritic cells can also be contacted with the inventive compositions using in vivo methods. In order to accomplish this, the CpGs are administered in combination with the VLP coupled to or mixed with antigens and the additional TLR ligand directly to a subject in need of immunotherapy. may be preferred that the VLPs/CpGs be administered in the local region of the tumor, which can be accomplished in any way known in the art, *e.g*., direct injection into the tumor.

In a further very preferred embodiment of the present invention, the unmethylated CpG-containing oligonucleotide comprises, or alternatively consists essentially of, or alternatively consists of the sequence GGGGGGGGGGGACGATCGTCGGGGGGGGGG (SEQ ID NO: 54). The latter was previously found to be able to stimulate blood cells in vitro (Kuramoto E. et al., Japanese Journal Cancer Research 83, 1128-1131 (1992).

In another preferred embodiment of the present invention, the immunostimulatory nucleic acid is an unmethylated CpG-containing oligonucleotide, wherein the CpG motif of said unmethylated CpG-containing oligonucleotide is part of a palindromic sequence. Preferably said palindromic sequence is GACGATCGTC (SEQ ID NO: 39). In another preferred embodiment, the palindromic sequence is flanked at its 3'-terminus and at its 5'-terminus by less than 10 guanosine entities, wherein preferably said palindromic sequence is GACGATCGTC (SEQ ID NO: 39). In a further preferred embodiment the palindromic sequence is flanked at its N-terminus by at least 3 and at most 9 guanosine entities and wherein said palindromic sequence is flanked at its C-terminus by at least 6 and at most 9 guanosine entities. These immunostimulatory nucleic acids have unexpectedly been found to be very efficiently packaged into VLPs. The packaging ability was hereby enhanced as compared to the corresponding immunostimulatory nucleic acid having the sequence GACGATCGTC (SEQ ID NO: 39) flanked by 10 guanosine entitites at the 5' and 3' terminus.

In a preferred embodiment of the present invention, the palindromic sequence comprises, or alternatively consist essentially of, or alternatively consists of or is GACGATCGTC (SEQ ID NO: 39), wherein said palindromic sequence is flanked at its 5'-terminus by at least 3 and at most 9 guanosine entities and wherein said palindromic sequence is flanked at its 3'-terminus by at least 6 and at most 9 guanosine entities.

In a further very preferred embodiment of the present invention, the immunostimulatory nucleic acid is an unmethylated CpG-containing oligonucleotide, wherein the CpG motif of said unmethylated CpG-containing oligonucleotide is part of a palindromic sequence, wherein said unmethylated CpG-containing oligonucleotide has a nucleic acid sequence selected from the group consisting of (a) GGGGACGATCGTCGGGGGG ((SEQ ID NO: 40); and typically abbreviated herein as G3-6), (b) GGGGGACGATCGTCGGGGGG ((SEQ ID NO: 41); and typically abbreviated herein as G4-6), (c) GGGGGGACGATCGTCGGGGGG ((SEQ ID NO: 42); and typically abbreviated herein as G5-6), (d) GGGGGGGACGATCGTCGGGGGG ((SEQ ID NO: 43); and typically abbreviated herein as G6-6), (e) GGGGGGGGACGATCGTCGGGGGGG ((SEQ ID NO: 44); and typically abbreviated herein as G7-7), (f) GGGGGGGGGACGATCGTCGGGGGGGG ((SEQ ID NO: 45); and typically abbreviated herein as G8-8), (g) GGGGGGGGGGACGATCGTCGGGGGGGGG ((SEQ ID NO: 46); and typically abbreviated herein as G9-9), and (h) GGGGGGCGACGACGATCGTCGTCGGGGGGG ((SEQ ID NO: 47); and typically abbreviated herein as G6).

In a further preferred embodiment of the present invention the immunostimulatory nucleic acid is an unmethylated CpG-containing oligonucleotide, wherein the CpG motif of said unmethylated CpG-containing oligonucleotide is part of a palindromic sequence, wherein said palindromic sequence is GACGATCGTC (SEQ IDNO: 39), and wherein said palindromic sequence is flanked at its 5'-terminus of at least 4 and at most 9 guanosine entities and wherein said palindromic sequence is flanked at its 3'-terminus of at least 6 and at most 9 guanosine entities.

In a further preferred embodiment of the present invention the immunostimulatory nucleic acid is an unmethylated CpG-containing oligonucleotide, wherein the CpG motif of said unmethylated CpG-containing oligonucleotide is part of a palindromic sequence, wherein said palindromic sequence is GACGATCGTC (SEQ ID NO: 39), and wherein said palindromic sequence is flanked at its 5'-terminus of at least 5 and at most 8 guanosine entities and wherein said palindromic sequence is flanked at its 3'-terminus of at least 6 and at most 8 guanosine entities.

The experimental data show that the ease of packaging of the preferred inventive immunostimulatory nucleic acids, i.e. the guanosine flanked, palindromic and unmethylated CpG-containing oligonucleotides, wherein the palindromic sequence is GACGATCGTC (SEQ ID NO: 39), and wherein the palindromic sequence is flanked at its 3'-terminus and at its 5'-terminus by less than 10 guanosine entities, into VLP's increases if the palindromic sequences are flanked by fewer guanosine entities. However, decreasing the number of guanosine entities flanking the palindromic sequences leads to a decrease of stimulating blood cells in vitro. Thus, packagability is paid by decreased biological activity of the indicated inventive immunostimulatory nucleic acids. The preferred embodiments represent, thus, a compromise between packagability and biological activity.

In a further preferred embodiment of the present invention the immunostimulatory nucleic acid is an unmethylated CpG-containing oligonucleotide, wherein the CpG motif of said unmethylated CpG-containing oligonucleotide is part of a palindromic sequence, wherein said unmethylated has the nucleic acid sequence of SEQ ID NO: 45, i.e.the immunostimulatory nucleic acid is G8-8.

Disclosed is a composition which comprises (a) a VLP, (b) at least one immunostimulatory nucleic acid, (c) at least one antigen, and (d) at least one TLR ligand, wherein said immunostimulatory nucleic acid (b) is an unmethylated CpG-containing oligonucleotide and wherein said ligand (d) is a ligand for TLR 1, 2, 3, 4, 5, 6, 7, 8, 10 or 11, and wherein said ligand for a TLR activates at least one TLR selected from the group consisting of TLR 1, 2, 3, 4, 5, 6, 7, 8, 10 and 11.

Further disclosed is a composition which comprises (a) a VLP, (b) at least one immunostimulatory nucleic acid, (c) at least one antigen, and (d) at least one TLR ligand, wherein said immunostimulatory nucleic acid (b) is poly (I:C), and wherein said ligand (d) is a ligand for TLR 1, 2, 4, 5, 6, 7, 8, 9, 10 or 11, and wherein said ligand for a TLR activates at least one TLR selected from the group consisting of TLR 1, 2, 4, 5, 6, 7, 8, 9, 10 and 11.

The inventive composition further comprises an antigen or antigenic determinant coupled with the modified virus-like particle. The invention provides for compositions that vary according to the antigen or antigenic determinant selected in consideration of the desired therapeutic effect. Antigens or antigenic determinants suitable for use in the present invention are disclosed in WO 00/32227, in WO 01/85208 and in WO 02/056905.

The antigen can be any antigen of known or yet unknown provenance. It can be isolated from bacteria; viruses or other pathogens; tumors; or trees, grass, weeds, plants, fungi, mold, dust mites, food, or animals known to trigger allergic responses in sensitized patients. Alternatively, the antigen can be a recombinant antigen obtained from expression of suitable nucleic acid coding therefor. In a preferred embodiment, the antigen is a recombinant antigen. The selection of the antigen is, of course, dependent upon the immunological response desired and the host.

The present invention is applicable to a wide variety of antigens, the antigen is may be a protein, polypeptide or peptide.

Antigens of the invention can be selected from the group consisting of the following: (a) polypeptides suited to induce an immune response against cancer cells; (b) polypeptides suited to induce an immune response against infectious diseases; (c) polypeptides suited to induce an immune response against allergens; (d) polypeptides suited to induce an immune response in farm animals or pets; (e) carbohydrates naturally present on the polypeptides and (f) fragments (*e.g*., a domain) of any of the polypeptides set out in (a)-(e).

Preferred antigens include those from a pathogen (*e.g*. virus, bacterium, parasite, fungus) tumors (especially tumor-associated antigens or "tumor markers") and allergens. Other preferred antigens are autoantigens and self antigens, respectively..

In some Examples, VLPs containing peptide p33 were used. It should be noted that the VLPs containing peptide p33 were used only for reasons of convenience, and that wild-type VLPs can likewise be used in the present invention. The peptide p33 derived from lymphocytic choriomeningitis virus (LCMV). The p33 peptide represents one of the best studied CTL epitopes. p33-specific T cells have been shown to induce lethal diabetic disease in transgenic mice (Speiser et al., J. Exp. Med 186:645 (1997)). This specific epitope, therefore, is particularly well suited to study autoimmunity, tumor immunology as well as viral diseases.

The antigen or antigenic determinant is one that may be useful for the prevention of infectious disease. Such treatment will be useful to treat a wide variety of infectious diseases affecting a wide range of hosts, *e.g*., human, cow, sheep, pig, dog, cat, other mammalian species and non-mammalian species as well. Infectious diseases are well known to those skilled in the art, and examples include infections of viral etiology such as HIV, influenza, *Herpes,* viral hepatitis, Epstein Bar, polio, viral encephalitis, measles, chicken pox, Papilloma virus etc.; or infections of bacterial etiology such as pneumonia, tuberculosis, syphilis, etc.; or infections of parasitic etiology such as malaria, trypanosomiasis, leishmaniasis, trichomoniasis, amoebiasis, etc. Thus, antigens or antigenic determinants selected for the compositions of the invention will be well known to those in the medical art; examples of antigens or antigenic determinants include the following: the HIV antigens gp140 and gp160; the influenza antigens hemagglutinin, M2 protein and neuraminidase, Hepatitis B surface antigen or core and circumsporozoite protein of malaria or fragments thereof.

As discussed above, antigens include infectious microbes such as viruses, bacteria and fungi and fragments thereof, derived from natural sources or synthetically.

Infectious microbes such as viruses, examples of RNA viruses, or illustrative DNA viruses that are antigens in vertebrate animals and that can be used for the composition of the present invention are desribed for example in WO 03/024481 (in particular on page 86 to 89)

The antigen may comprise one or more cytotoxic T cell epitopes, Th cell epitopes, or a combination of cytotoxic T cell epitopes and Th cell epitopes.

In addition to enhancing an antigen specific immune response in humans, the methods of the preferred embodiments are particularly well suited for treatment of other mammals or other animals, *e.g*., birds such as hens, chickens, turkeys, ducks, geese, quail and pheasant. Birds are prime targets for many types of infections. Other examples of antigens that can be used for the composition of the present invention are described in WO 03/024481 (page 90 to 93).

In another aspect there is provided vaccine compositions suitable for use in methods for preventing and/or attenuating diseases or conditions which are caused or exacerbated by "self' gene products (*e.g*., tumor necrosis factors). Thus, vaccine compositions include compositions which lead to the production of antibodies that prevent and/or attenuate diseases or conditions caused or exacerbated by "self' gene products. Examples of such diseases or conditions include graft versus host disease, IgE-mediated allergic reactions, anaphylaxis, adult respiratory distress syndrome, Crohn's disease, allergic asthma, acute lymphoblastic leukemia (ALL), non-Hodgkin's lymphoma (NHL), Graves' disease, systemic lupus erythematosus (SLE), inflammatory autoimmune diseases, myasthenia gravis, immunoproliferative disease lymphadenopathy (IPL), angioimmunoproliferative lymphadenopathy (AIL), immunoblastive lymphadenopathy (IBL), rheumatoid arthritis, diabetes, multiple sclerosis, Alzheimer disease and osteoporosis.

Compositions of the invention are may be an immunotherapeutic that can be used for the treatment and/or prevention of allergies, cancer or drug addiction.

The selection of antigens or antigenic determinants for the preparation of compositions and for use in methods of treatment for allergies would be known to those skilled in the medical arts treating such disorders. Representative examples of such antigens or antigenic determinants include the following: bee venom phospholipase A₂; Amb a 1 (ragweed pollen allergen), Bet v I (birch pollen allergen); 5 Dol m V (white-faced hornet venom allergen); Der p 1, Der f 2 and Der 2 (house dust mite allergens); Lep d 2 (dust mite allergen); Alt a 1, Asp f1, and Asp f 16 (fungus allergens); Ara h 1, Ara h 2, and Ara h3 (peanut allergens) as well as fragments of each which can be used to elicit immunological responses. Moreover, this may be particularly useful for the use of allergen mixtures that have been isolated from organisms or parts of organisms, such as pollen extracts or bee venom.

In a preferred embodiment, pollen extracts comprise, or alternatively consist of trees, grasses, weeds, and garden plants. Examples of tree pollen extracts include, but are not limited to, the following: acacia, alder (grey), almond, apple, apricot, arbor vitae, ash, aspen, bayberry, beech, birch (spring), birch (white), bottle brush, box elder, carob tree, cedar, including but not limited to the japanese cedar, cherry, chestnut, cottonwood, cypress, elderberry, elm (American), eucalyptus, fir, hackberry, hazelnut, hemlock, hickory, hop-hornbeam, ironwood, juniper, locust, maple, melaleuca, mesquite, mock orange, mulberry, oak (white), olive, orange, osage orange, palo verde, peach, pear, pecan, pepper tree, pine, plum, poplar, privet, redwood, Russian olive, spruce, sweet gum, sycamore, tamarack, tree of heaven, walnut and willow. Examples of grass pollen extracts include, but are not limited to, the following: bahia, barley, beach, bent, Bermuda grass, bluegrass (Kentucky), brome, bunch, canarygrass, chess, corn, fescue (meadow), grama, johnson, june grass, koeler's, oats, orchard grass, quack, redtop, rye grass (perennial), salt, sorghum, sudan, sweet vernal grass, timothy grass, velvetgrass, wheat and wheatgrass. Examples of weed and garden plant extracts include, but are not limited to, the following: alfalfa, amaranth, aster, balsam root, bassia, beach bur, broomwood, burrow bush, careless weed, castor bean, chamise, clover, cocklebur, coreopsis, cosmos, daffodil, dahlia, daisy, dandelion, dock, dog fennel, fireweed, gladiolus, goldenrod, greasewood, hemp, honeysuckle, hops, iodone bush, Jerusalem oak, kochia, lamb's quarters, lily, marigold, marshelder, Mexican tea, mugwort, mustard, nettle, pickleweed, pigweed, plaintain (English), poppy, povertyweed, quailbush, ragweed (giant), ragweed (short), ragweed (western), rose, Russian thistle, sagebrush, saltbrush, scale, scotch broom, sea blight, sheep sorrel, snapdragon, sugar beet, sunflower, western waterhemp, winter fat, wormseed, wormwood.

Pollen extracts may comprise, or alternatively consist of rye.

The seasonal appearance of ragweed pollen (September-October) induces asthma in many individuals (Marshall, J. et al., J. Allergy Clin Immunol.108:191-197 (2001)). Asthma is characterized by pulmonary inflammation, reversible airflow obstruction, and airway hyperresponsivess. A complex cascade of immunological responses to aeroallergens leads to leukocyte recruitment in the airways. Specifically, lymphocytes, macrophages, eosinophils, neutrophils, plasma cells, and mast cells infiltrate the bronchial mucosa (Redman, T. et al., Exp. Lung Res. 27:433-451 (2001)). Eosinophil recruitment is associated with increased production of the T2 cytokines IL-4 and IL-5, key factors in asthma pathogenesis that support the chronic inflammatory process (Justice, J. et al., Am. J. Physiol. Lung Cell Mol. Physiol. 282:L302-L309 (2002) ). The immunodominant ragweed allergen in short ragweed (*Ambrosia artemisiifolia*) is Amb a 1 (Santeliz, J. et al., J. Allergy Clin. Immunol. 109:455-462 (2002)). The composition may comprise the Amb a 1 mixed with or coupled to the virus-like particle.

Dust extracts may comprise, or alternatively consist of house dusts and dust mites. Examples of house dusts include, but are not limited to: house dust, mattress dust, and upholstrey dust. Examples of dust mites include, but are not limited to, *D. farniae, D. ptrerorrysiinus,* mite mix, and *L. destructor.* Dust extracts also include, but are not limited to, cedar and red cedar dust, cotton gin dust, oak dust, grain (elevator) dust, paduk dust and wood dust.

Dust mites are an important source of perennial indoor allergens in homes in humid climates of developed countries (Arlian, L., Current Allergy and Asthma Reports 1:581-586 (2001)). About 60-85% of all patients with allergic bronchial asthma are sensitized to the house dust mite *Dermatophogoldes pteronyssinus* (Arlian, L., Current Allergy and Asthma Reports 1:581-586 (2001)). Immunodominant *D. pteronyssinus* dust mite allergens include Der p 1, Der f2, and Der 2 (Kircher, M. et al., J. Allergy Clin. Immunol. 109:517-523 (2002) and Clarke, A. et al., Int. Arch. Allergy Immunol. 120:126-134 (1999) The composition may comprise the Der p 1, Der f 2, Der 2, or fragments thereof, or an antigenic mixture thereof coupled to or mixed with the virus-like particle. An important cause of allergic reactions to dust, especially in farming communities, is *Lepidoglyphus destructor* (Ericksson, T. et al., Clinical and Exp. Allergy 31:1181-1890 (2001)). An immunodominant *L. destructor* dust mite allergen is Lep d 2 (Ericksson, T. et al., Clinical and Exp. Allergy 31:1181-1890 (2001)). The composition comprises the Lep d 2 coupled to or mixed with the virus-like particle.

Fungal extracts may comprise, or alternatively consist of alternaria, aspergillus, botrytis, candida, cephalosporium, cephalothecium, chaetomium, cladosporium, crytococcus, curvularia, epicoccum, epidermophyton, fusarium, gelasinospora, geotrichum, gliocladium, helminthosporium, hormodendrum, microsporium, mucor, mycogone, nigraspora, paecilomyces, penicillium, phoma, pullularia, rhizopus, rhodotorula, rusts, saccharomyces, smuts, spondylocladium, stemphylium, trichoderma, trichophyton and verticillium.

*Alternaria alternata* is considered to be one of the most important fungi causing allergic disease in the United States. *Alternaria* is the major asthma-associated allergen in desert regions of the United States and Australia and has been reported to cause serious respiratory arrest and death in the US Midwest (Vailes, L. et al., J. Allergy Clin. Immunol. 107:641 (2001) and Shampain, M. et al., Am. Rev. Respir. Dis. 126:493-498 (1982) . The immunodominant *Alternaria alternata* antigen is Alt a 1 (Vailes, L. et al., J. Allergy Clin. Immunol. 107:641 (2001)). Greater than 80% of *Alternaria* sensitized individuals have Ig E antibody against Alt a 1 (Vailes, L. et al., Clinical and Exp. Allergy 31:1891-1895 (2001)). In a specific embodiment of the invention, the composition comprises the Alt a 1 coupled to or mixed with the virus-like particle.

Another opportunistic fungi is *Aspergillus fumigatus,* which is involved in a broad spectrum of pulmonary diseases, including allergic asthma. Immunodominant *Aspergillus fumigatus* antigens include Asp f I and Asp f 16 (Vailes, L. et al., J. Allergy Clin. Immunol. 107:641 (2001)). In a specific embodiment of the invention, the composition comprises the Asp f1 or Asp f16 or an antigenic mixture thereof coupled to or mixed with the virus-like particle.

Insect extracts may comprise, or alternatively consist of, stinging insects whose whole body induces allergic reactions, stinging insects whose venom protein induces allergic reactions, and insects that induce inhaled allergic reactions. Examples of stinging insects whose whole body induces allergic reactions include, but are not limited to: ant (black), ant (red), ant (carpenter), ant mix (black/red), ant (fire). Examples of stinging insects whose venom protein induces allergic reactions include, but are not limited to: honey bee, yellow hornet, wasp, yellow jacket, white-faced hornet and mixed vespid. Examples of insects that induce inhaled allergic reactions include, but are not limited to: aphid, black fly, butterfly, caddis fly, cicada/locust, cricket, cockroach, daphnia, deerfly, fruit fly, honey bee (whole body), horse fly, house fly, leafhopper, may fly, Mexican bean weevil, mites (dust), mosquito, moth, mushroom fly, screwworm fly, sow bugs, spider and water flea.

Food extracts comprise, or alternatively consist of, animal products and plant products. Examples of animal products include, but are not limited to: beef, chicken, deer, duck, egg (chicken), fish, goat, goose, lamb, milk (cow), milk (goat), pork, rabbit, shellfish and turkey. Examples of plant products include, but are not limited to: apple, apricot, arrowroot, artichoke, asparagus, avodaco, banana, bean, beet, berries, cabbage family, carrot, celery, cherry, chocolate, citrus fruits, coconut, coffee, cucumber, date, eggplant, grain, grape, greens, gums, hops, lettuce, malt, mango, melon, mushroom, nuts, okra, olive, onion, papaya, parsnip, pea, peanut, pear, pimento, pineapple, plum, potato, prune, pumpkin, radish, rhubarb, spice%ondiment, spinach, squash, tapioca, tea, tomato, watermelon and yeast.

Two major allergenic peanut proteins, which are recognized by more than 95% of patients with peanut allergy, are Ara h1 and Ara h 2 (Bannon, G., et al., Int. Arch. Allergy Immunol. 124:70-72 (2001) and Li, X. et al., J. Allergy Clin. Immunol. 106:150-158 (2000). Ara h 3 is recognized by about 45% of patients with peanut allergy (Li, X., et al., J Allergy Clin. Immunol. 106:150-158 (2000)). In a specific embodiment of the invention, the composition comprises the antigen Ara h 1, Ara h 2, or Ara h 3 or an antigenic mixture thereof coupled to or mixed with the virus-like particle.

Mammalian epidermal allergens may include, but are not limited to: camel, cat hair, cat pelt, chinchilla, cow, deer, dog, gerbil, goat, guinea pig, hamster, hog, horse, mohair, monkey, mouse, rabbit, wool (sheep). In yet another preferred embodiment, feathers include, but are not limited to: canary, chicken, duck, goose, parakeet, pigeon, turkey. Other inhalants include, but are not limited to: acacia, algae, castor bean, cotton linters, cottonseed, derris root, fern spores, grain dusts, hemp fiber, henna, flaxseed, guar gum, jute, karaya gum, kapok, leather, lycopodium, orris root, pyrethrum, silk (raw), sisal, tobacco leaf, tragacanth and wood dusts.

Typically defined mammalian allergens, either purified from natural sources or recombinantly expressed are included. These include, but are not limited, to Fel d 1, Fel d 3 (cystatin) from cats and albumins from cat, camel, chinchilla, cow, deer, dog, gerbil, goat, guinea pig, hamster, hog, horse, mohair, monkey, mouse, rabbit, wool (sheep).

The selection of antigens or antigenic determinants for compositions and methods of treatment for cancer would be known to those skilled in the medical arts treating such disorders (see Renkvist et al., Cancer. Immunol. Immunother. 50:3-15 (2001) and such antigens or antigenic determinants are included within the scope of the present invention. Representative examples of such types of antigens or antigenic determinants include the following: Her2 (breast cancer); GD2 (neuroblastoma); EGF-R (malignant glioblastoma); CEA (medullary thyroid cancer); CD52 (leukemia); human melanoma protein gp100; human melanoma protein gp 100 epitopes such as amino acids 154-162 (sequence: KTWGQYWQV, SEQ ID NO: 14), 209-217 (ITDQVPFSV, SEQ ID NO: 15), 280-288 (YLEPGPVTA, SEQ ID NO: 16), 457-466 (LLDGTATLRL, SEQ ID NO: 17) and 476-485 (VLYRYGSFSV, SEQ ID NO: 18); human melanoma protein melan-A/MART-1; human melanoma protein melan-A/MART-1 epitopes such as amino acids 26-35 (EAAGIGILTV) (SEQ ID NO:37), 26-35AL (ELAGIGICTV, SEQ ID NO: 38), 27-35 (AAGIGILTV, SEQ ID NO: 19) and 32-40 (ILTVILGVL, SEQ ID NO: 20); tyrosinase and tyrosinase related proteins (*e.g*., TRP-1 and TRP-2); tyrosinase epitopes such as amino acids 1-9 (MLLAVLYCL, SEQ ID NO: 21) and 368-376 (YMDGTMSQV, SEQ ID NO: 22); NA17-A nt protein; NA17-A nt protein epitopes such as amino acids 38-64 (VLPDVFIRC, SEQ ID NO: 23); MAGE-3 protein; MAGE-3 protein epitopes such as amino acids 271-279 (FLWGPRALV, SEQ ID NO: 24); other human tumors antigens, *e.g.* CEA epitopes such as amino acids 571-579 (YLSGANLNL, SEQ ID NO: 25); p53 protein; p53 protein epitopes such as amino acids 65-73 (RMPEAAPPV, SEQ ID NO: 26), 149-157 (STPPPGTRV, SEQ ID NO: 27) and 264-272 (LLGRNSFEV, SEQ ID NO: 28); Her2/neu epitopes such as amino acids 369-377 (KIFGSLAFL, SEQ ID NO: 29) and 654-662 (IISAVVGIL, SEQ ID NO: 30); HPV 16 E7 protein; HPV 16 E7 protein epitopes such as amino acids 86-93 (TLGIVCPI, SEQ ID NO: 31); as well as fragments or mutants of each which can be used to elicit immunological responses.

The selection of antigens or antigenic determinants for compositions and methods of treatment for other diseases or conditions associated with self antigens would be also known to those skilled in the medical arts treating such disorders. Representative examples of such antigens or antigenic determinants are, for example, lymphotoxins (*e.g*. Lymphotoxin α (LT α), Lymphotoxin β (LT β)), and lymphotoxin receptors, Receptor activator of nuclear factor kappaB ligand (RANKL), Osteoclast-associated receptor (OSCAR), vascular endothelial growth factor (VEGF) and vascular endothelial growth factor receptor (VEGF-R), Interleukin 17 and amyloid beta peptide (Aβ₁₋₄₂), TNFα, MIF, MCP-1, SDF-1, Rank-L, M-CSF, Angiotensinogen, Angiotensin I, Angiotensin II, Endoglin, Eotaxin, Grehlin, BLC, CCL21, IL-13, IL-17, IL-5, IL-8, IL-15, Bradykinin, Resistin, LHRH, GHRH, GIH, CRH, TRH and Gastrin, as well as fragments of each which can be used to elicit immunological responses.

The antigen or antigenic determinant may be selected from the group consisting of: (a) a recombinant polypeptide of HIV; (b) a recombinant polypeptide of Influenza virus (*e.g*., an Influenza virus M2 polypeptide or a fragment thereof); (c) a recombinant polypeptide of Hepatitis C virus; (d) a recombinant polypeptide of Hepatitis B virus; (e) a recombinant polypeptide of *Toxoplasma;* (f) a recombinant polypeptide of *Plasmodium falciparum;* (g) a recombinant polypeptide of *Plasmodium vivax;* (h) a recombinant polypeptide of *Plasmodium ovale;* (i) a recombinant polypeptide of *Plasmodium malariae;* (j) a recombinant polypeptide of breast cancer cells; (k) a recombinant polypeptide of kidney cancer cells; (1) a recombinant polypeptide of prostate cancer cells; (m) a recombinant polypeptide of skin cancer cells; (n) a recombinant polypeptide of brain cancer cells; (o) a recombinant polypeptide of leukemia cells; (p) a recombinant profiling; (q) a recombinant polypeptide of bee sting allergy; (r) a recombinant polypeptide of nut allergy; (s) a recombinant polypeptide of pollen; (t) a recombinant polypeptide of house-dust; (u) a recombinant polypeptide of cat or cat hair allergy; (v) a recombinant protein of food allergies; (w) a recombinant protein of asthma; (x) a recombinant protein of *Chlamydia;* (y) antigens extracted from any of the protein sources mentioned in (a-x); and (z) a fragment of any of the proteins set out in (a)-(x).

The antigen coupled to the virus-like particle packaged with the immunostimulatory nucleic acid, namely the unmethylated CpG-containing oligonucleotide of the invention may be a T cell epitope, either a cytotoxic or a Th cell epitope. The antigen coupled with the virus-like particle packaged with the immunostimulatory nucleic acid or preferably the unmethylated CpG-containing oligonucleotide of the invention may also be a B cell epitope. The antigen may be a combination of at least two, preferably different, epitopes, wherein the at least two epitopes are linked directly or by way of a linking sequence. These epitopes are preferably selected from the group consisting of cytotoxic and Th cell epitopes.

The antigen and in particular the indicated epitope or epitopes, can be synthesized or recombinantly expressed and coupled to the virus-like particle, or fused to the virus-like particle using recombinant DNA techniques. Exemplary procedures describing the attachment of antigens to virus-like particles are disclosed in WO 00/32227, in WO 01/85208 and in WO 02/056905.

Vaccine compositions which can be used for preventing and/or attenuating diseases or conditions are also disclosed. Vaccine compositions comprise, or alternatively consist of, an immunologically effective amount of the inventive immune enhancing composition together with a pharmaceutically acceptable diluent, carrier or excipient.

Further disclosed are vaccination methods for preventing and/or attenuating diseases or conditions in animals. Disclosed are vaccines for the prevention of infectious diseases in a wide range of animal species, particularly mammalian species such as human, monkey, cow, dog, cat, horse, pig, etc. Vaccines can be designed to treat infections of viral etiology such as HIV, influenza, *Herpes,* viral hepatitis, Epstein Bar, polio, viral encephalitis, measles, chicken pox, etc.; or infections of bacterial etiology such as pneumonia, tuberculosis, syphilis, etc.; or infections of parasitic etiology such as malaria, trypanosomiasis, leishmaniasis, trichomoniasis, amoebiasis, etc.

Further disclosed are, vaccines for the prevention of cancer in a wide range of species, particularly mammalian species such as human, monkey, cow, dog, cat, horse, pig, etc. Vaccines can be designed to treat all types of cancer including, but not limited to, lymphomas, carcinomas, sarcomas and melanomas.

As would be understood by one of ordinary skill in the art, when compositions of the invention are administered to an animal, they can be in a composition which contains salts, buffers, adjuvants or other substances which are desirable for improving the efficacy of the composition. Examples of materials suitable for use in preparing pharmaceutical compositions are provided in numerous sources including REMINGTON'S PHARMACEUTICAL SCIENCES (Osol, A, ed., Mack Publishing Co., (1990)).

Compositions of the invention are said to be "pharmacologically acceptable" if their administration can be tolerated by a recipient individual. Further, the compositions of the invention will be administered in a "therapeutically effective amount" (*i.e*., an amount that produces a desired physiological effect).

The compositions of the present invention can be administered by various methods known in the art. The particular mode selected will depend of course, upon the particular composition selected, the severity of the condition being treated and the dosage required for therapeutic efficacy. The compositions of the invention, generally speaking, can administered using any mode that is medically acceptable, meaning any mode that produces effective levels of the active compounds without causing clinically unacceptable adverse effects. Such modes of administration include oral, rectal, parenteral, intracistemal, intravaginal, intraperitoneal, topical (as by powders, ointments, drops or transdermal patch), bucal, or as an oral or nasal spray. The term "parenteral" as used herein refers to modes of administration which include intravenous, intramuscular, intraperitoneal, intrasternal, subcutaneous and intraarticular injection and infusion. The composition of the invention can also be injected directly in a lymph node.

Components of compositions for administration include sterile aqueous (*e.g*., physiological saline) or non-aqueous solutions and suspensions. Examples of non-aqueous solvents are propylene glycol, polyethylene glycol, vegetable oils such as olive oil, and injectable organic esters such as ethyl oleate. Carriers or occlusive dressings can be used to increase skin permeability and enhance antigen absorption.

Combinations can be administered either concomitantly, *e.g*., as an admixture, separately but simultaneously or concurrently; or sequentially. This includes presentations in which the combined agents are administered together as a therapeutic mixture, and also procedures in which the combined agents are administered separately but simultaneously, *e.g*., as through separate intravenous lines into the same individual. Administration "in combination" further includes the separate administration of one of the compounds or agents given first, followed by the second.

Dosage levels depend on the mode of administration, the nature of the subject, and the quality of the carrier/adjuvant formulation. Typical amounts for VLPs, antigen and adjuvants are in the range of about 0.001 µg to about 20 mg per subject. Preferred amounts are at least about 10 µg to about 500 µg per subject. Multiple administration to immunize the subject is preferred, and protocols are those standard in the art adapted to the subject in question. Typical amounts of the antigen are in a range comparable, similar or identical to the range typically used for administration without the addition of the VLP's.

The compositions can conveniently be presented in unit dosage form and can be prepared by any of the methods well-known in the art of pharmacy. Methods include the step of bringing the compositions of the invention into association with a carrier which constitutes one or more accessory ingredients. In general, the compositions are prepared by uniformly and intimately bringing the compositions of the invention into association with a liquid carrier, a finely divided solid carrier, or both, and then, if necessary, shaping the product.

Compositions suitable for oral administration can be presented as discrete units, such as capsules, tablets or lozenges, each containing a predetermined amount of the compositions of the invention. Other compositions include suspensions in aqueous liquids or non-aqueous liquids such as syrup, an elixir or an emulsion.

Other delivery systems can include time-release, delayed release or sustained release delivery systems. Such systems can avoid repeated administrations of the compositions of the invention described above, increasing convenience to the subject and the physician. Many types of release delivery systems are available and known to those of ordinary skill in the art.

Further disclosed are processes for the production of the compositions of the invention and methods of medical treatment for cancer and allergies using said compositions.

Thus, the present invention, inter alia, relates to the finding that virus like particles (VLPs) loaded and packaged, respectively, with DNA oligonucleotides rich in non-methylated C and G (CpGs) together with a TLR-4 ligand, and antigens coupled to with the VLP, induce enhanced immune response against these antigens. Suprisingly, the immunogenicity was dramatically enhanced, if a TLR-4 ligand was added to the composition. In addition, the T cell responses against the antigens are especially directed to the Th1 type.

The following examples are illustrative only and are not intended to limit the scope of the invention as defined by the appended claims.

### EXAMPLE I

### Generation of VLPs

The DNA sequence of HBcAg containing peptide p33 from LCMV is given in SEQ ID NO: 12. The p33-HBcAg VLPs (p33-VLPs) were generated as follows: Hepatitis B clone pEco63 containing the complete viral genome of Hepatitis B virus was purchased from ATCC. The generation of the expression plasmid has been described previously (*see* WO 03/024481).

A clone of E. coli K802 selected for good expression was transfected with the plasmid, and cells were grown and resuspended in 5 ml lysis buffer (10 mM Na₂HPO₄, 30 mM NaCl, 10 mM EDTA, 0.25 % Tween-20, pH 7.0). 200 µl of lysozyme solution (20 mg/ml) was added. After sonication, 4 µl Benzonase and 10 mM MgCl₂ was added and the suspension was incubation for 30 minutes at RT, centrifuged for 15 minutes at 15,000 rpm at 4°C and the supernatant was retained.

Next, 20 % (w/v) (0.2 g/ml lysate) ammonium sulfate was added to the supernatant. After incubation for 30 minutes on ice and centrifugation for 15 minutes at 20,000 rpm at 4°C the supernatant was discarded and the pellet resuspended in 2-3 ml PBS. 20 ml of the PBS-solution was loaded onto a Sephacryl S-400 gel filtration column (Amersham Pharmacia Biotechnology AG), fractions were loaded onto a SDS-Page gel and fractions with purified p33-HBcAg VLP capsids were pooled. Pooled fractions were loaded onto a Hydroxyappatite column. Flow through (which contains purified p33-HBcAg VLP capsids) was collected. Electron microscopy was performed according to standard protocols. A representative example is shown in Figure 1 of Storni T., et al.,(2002) J Immunol.; 168(6):2880-6.

It should be noted that the VLPs containing peptide p33 were used only for reasons of convenience, and that wild-type VLPs can likewise be used in the present invention. Throughout the description the terms p33-HBcAg VLP, HBcAg-p33 VLP, p33-VLPs and HBc33 are used interchangeably.

### EXAMPLE 2

CpG-containing oligonucleotides can be packaged into HBcAg VLPs.

Recombinant VLPs generated as described in Example 1 were run on a native agarose (1%) gel electrophoresis and stained with ethidium bromide or Coomassie blue for the detection of RNA/DNA or protein (Fig. 1). Bacterial produced VLPs contain high levels of single stranded RNA, which is presumably binding to the arginine repeats appearing near the C-terminus of the HBcAg protein and being geographically located inside the VLPs as shown by X-ray crystallography. The contaminating RNA can be easily digested and so eliminated by incubating the VLPs with RNase A. The highly active RNase A enzyme has a molecular weight of about 14 kDa and is presumably small enough to enter the VLPs to eliminate the undesired ribonucleic acids.

The recombinant VLPs were supplemented with CpG-rich oligonucleotides (see SEQ ID NO: 11) before digestion with RNase A. As shown in Fig. 2 the presence of CpG- oligonucleotides preserved the capsids structure as shown by similar migration compared to untreated p33-VLPs. The CpG- oligonucleotides containing VLPs were purified from unbound oligonucleotides via dialysis (4500-fold dilution in PBS for 24 hours using a 300 kDa MWCO dialysis membrane) (see Fig. 3).

### EXAMPLE 3

CpG-containing oligonucleotides can be packaged into VLPs by removal of the RNA with RNAse and subsequent packaging of oligonucleotides into VLPs.

The VLPs (containing bacterial single-stranded RNA and generated as described in Example 1) were first incubated with RNaseA to remove the RNA and in a second step the immunostimulating CpG-oligonucleotides (with normal phosphodiester moieties but also with phosphorothioate modifications of the phosphate backbone) was supplemented to the samples (Fig. 4). This experiment clearly shows that the CpG-oligonucleotides are not absolutely required simultaneously during the RNA degradation reaction but can be added at a later time.

### EXAMPLE 4

Packaging of CpG oligonucleotides into the RNA bacteriophage Qb by RNAse digestion.

VLPs formed by the coat protein of the RNA bacteriophage Qb was used for this experiment. They were used either untreated or after packaging with CpG-2006 oligonucleotides (SEQ-ID NO: 114) having phosphorothioate modifications of the phosphorus backbone. Packaging of CpG-2006 was achieved by incubating 8 ml of a Qb VLP solution (2.2 mg/ml) at 60°C overnight in the presence of 0.2 ml of a 100 mM ZnSO₄ solution. This treatment leads to hydrolysis of the RNA contained in the Qb VLPs. After dialysis against 20 mM Hepes, pH 7.5 using a dialysis tube (cut-off MWCO 300000), CpG-2006 was added at 130 nmol / 1 ml VLP solution and incubated for 3 h at 37°C under shaking at 650 rpm. Removal of unpackaged CpG-2006 was achieved by subsequent treatment with 50 U/ml Benzonase (Merck) for 3 h at 37°C in the presence of 1 mM MgCl₂ followed by a dialysis against 20 mM Hepes, pH 7.5 as discribed above. Packaging of CpG-2006 was verified by agarose gel electrophoresis stained with ethidium bromide for visualization of nucleic acids and subsequently with Coomassie Blue for visualization of protein. In addition packaged VLPs were analysed on TBE-urea gels and amounts of packaged CpG-oligonucleotides estimated. About 6.7 nmol of CpG-2006 were packaged in 100 ug Qb VLPs.

### EXAMPLE 5

### Packaging of immunostimulatory nucleic acids into VLPs.

### RNaseA and ZnSO₄ mediated degradation of the nucleic acid content of a VLP.

Qβ VLPs were treated with RNaseA under low ionic strength conditions (20 mM Hepes pH 7.4 or 4 mM Hepes, 30 mM NaCl, pH 7.4). Alternatively, Qβ VLPs and AP205 VLPs were treated with ZnSO₄ under low ionic strength conditions (20 mM Hepes pH 7.4 or 4 mM Hepes, 30 mM NaCl pH 7.4) similar as described in Example 11. AP205 VLP (1 mg/ml) in either 20 mM Hepes pH 7.4 or 20 mM Hepes, 1 mM Tris, pH 7.4 was treated for 48 h with 2.5 mM ZnSO4 at 50°C in an Eppendorf Thermomixer comfort at 550 rpm. Qβ and AP205 VLP samples were centrifuged at 14000 rpm and supernatants were dialysed in 10.000 MWCO Spectra/Por® dialysis tubing (Spectrum, Cat. nr. 128 118) against first 2120 mM Hepes, pH 7.4 for 2 h at 4°C and, after buffer exchange, overnight. Samples were clarified after dialysis similar as described in Example 4 and protein concentration in the supernatants was determined by Bradford analysis.

### Packaging of ISS into RnaseA and ZnSO₄ treated VLPs.

After RNA hydrolysis and dialysis, Qβ and AP205 VLPs (1-1.5 mg/ml) were mixed with 130 µl of CpG oligonucleotides (NKCpG - cf. Table 1; G3-6, G8-8 - cf.

Table 2; 1 mM oligonucleotide stock in 10 mM Tris pH 8) per ml of VLPs. Samples were incubated for 3 h at 37°C in a thermoshaker at 650 rpm. Subsequently, samples were treated with 125 U Benzonase/ml VLPs (Merck KGaA, Darmstadt, Germany) in the presence of 2 mM MgCl₂ and incubated for 3 h at 37°C before dialysis. Samples were dialysed in 300.000 MWCO Spectra/Por® dialysis tubing (Spectrum, Cat. nr. 131 447) against 20 mM Hepes, pH 7.4 for 2 h at 4°C, and after buffer exchange overnight against the same buffer. After dialysis samples were centrifuged at 14000 rpm and protein concentration in the supernatants were determined by Bradford analysis.

Agarose gel electrophoresis and subsequent staining with ethidium bromide and Coomassie Blue showed that oligonucleotides were packaged in the VLPs.

### EXAMPLE 6

### Packaging ribonucleic acid into VLPs.

### ZnSO₄ dependent degradation of the nucleic acid content of a VLP.

Qβ VLPs were treated with ZnSO₄ under low ionic strength conditions (20 mM Hepes pH 7.4 or 4 mM Hepes, 30 mM NaCl, pH 7.4) similar as described in Example 11. AP205 VLPs (1 mg/ml) in either 20 mM Hepes pH 7.4 or 20 mM Hepes, 1 mM Tris, pH 7.4 were treated for 48 h with 2.5 mM ZnSO4 at 50°C in an Eppendorf Thermomixer comfort at 550 rpm. Qβ and AP205 VLP samples were centrifuged at 14000 rpm and dialysed against 20 mM Hepes, pH 7.4 as in Example 8.

### Packaging of poly (I:C) into ZnSO₄-treated VLPs:

The immunostimulatory ribonucleic acid poly (I:C), (Cat. nr. 27-4732-01, poly(I)-poly(C), Pharmacia Biotech) was dissolved in PBS (Invitrogen cat. nr. 14040) or water to a concentration of 4 mg/ml (9µM). Poly (I:C) was incubated for 10 minutes at 60°C and then cooled to 37°C. Incubated poly (I:C) was added in a 10-fold molar excess to either ZnSO₄-treated Qβ or AP205 VLPs (1-1.5 mg/ml) and the mixtures were incubated for 3 h at 37°C in a thermomixer at 650 rpm. Subsequently, excess of free poly (I:C) was enzymatically hydrolysed by incubation with 125 U Benzonase per ml VLP mixture in the presence of 2 mM MgCl₂ for 3 h at 37°C in a thermomixer at 300 rpm. Upon Benzonase hydrolysis samples were centrifuged at 14000 rpm and supernatants were dialysed in 300.000 MWCO Spectra/Por® dialysis tubing (Spectrum, Cat. nr. 131 447) against 2120 mM Hepes, pH 7.4 for 2 h at 4°C, and after buffer exchange overnight against the same buffer.. After dialysis, samples were centrifuged at 14000 rpm and protein concentration in the supernatants were determined by Bradford analysis.

Packaging is confirmed on 1% agarose gels and, after proteinase K digestion, on TBE/urea gels.

### EXAMPLE 7

### Packaging ribonucleic acid into HBcAg VLPs.

HBcAg VLPs are treated with ZnSO₄ under low ionic strength conditions (20 mM Hepes pH 7.4 or 4 mM Hepes, 30 mM NaCl, pH 7.4) similar as described in Example 11 and are dialysed against 20 mM Hepes pH 7.4 as in Example 22. Poly (I:C) is added in a 10-fold molar excess to HBcAg VLPs (1-1.5 mg/ml) and incubated for 3 h at 37°C in a thermomixer at 650 rpm as described in Example 24. Subsequently, excess of free poly (I:C) is enzymatically hydrolysed by incubation with 125 U Benzonase per ml VLP mixture in the presence of 2 mM MgCl₂ for 3 h at 37°C in a thermomixer at 300 rpm. Samples are clarified after Benzonase hydrolysis similar as described in Example 4 and dialysed as in Example 9. After dialysis, samples are centrifuged at 14000 rpm and protein concentration in the supernatants are determined by Bradford analysis.

### EXAMPLE 8

### Only CpGs are able to enhance CTL responses against p33 fused to HBcAg (p33-VLPs).

The p33-VLPs were generated as follows: Hepatitis B clone pEco63 containing the complete viral genome of Hepatitis B virus was purchased from ATCC. The gene encoding HBcAg was introduced into the EcoRI/HindIII restriction sites of expression vector pKK223.3 (Amersham Pharmacia Biotech Inc., NJ) under the control of a tac promotor. The p33 peptide (KAVYNFATM) (SEQ ID NO: 60) derived from LCMV was fused to the C-terminus of HBcAg (aa 1-183) via a three leucine-linker by standard PCR methods. *E. coli* K802d were transfected with the plasmid and grown in 2 liter cultures until an optical density of 1 (at 600 nm wavelength). Cells were induced by adding IPTG (Sigma, Division of Fluka AG, Switzerland) to a final concentration of 1mM for 4 hours. Bacteria were then collected by centrifugation and resuspended in 5 ml lysis buffer (10 mM Na₂HPO₄, 30 mM NaCl, 10 mM EDTA, 0.25 % Tween-20, pH 7.0). 200 µl of lysozyme solution (20 mg/ml) was added. After sonication 4 µl benzonase (Merck, Darmstadt, Germany) and 10 mM MgCl₂ were supplemented to the cell lysate. The suspension was then incubated for 30 minutes at RT and centrifuged for 15 minutes at 27000 x g. The retained supernatant was complemented with 20 % (w/v) ammonium sulfate. After incubation for 30 minutes on ice and centrifugation for 15 minutes at 48000 x g the supernatant was discarded and the pellet resuspended in 2-3 ml phosphate-saline buffer. The praparation was loaded onto a Sephacryl S-400 gel filtration column (Amersham Pharmacia Biotech Inc., NJ) for purification. Fractions were analyzed for protein content in a SDS PAGE gel and samples containing pure HBc capsids were pooled. Electron microscopy was performed according to standard protocols. Mice were immunized with p33 VLPs (100 ug) in the presence of various TLR ligands (100 ug each) (See Fig. 5):

LPS (E.coli K-235) was purchased from Sigma (Buchs, Switzerland), Poly (I:C) from Amersham Biosciences (Dübendorf, Switzerland), Peptidoglycan (S. aureus) from Fluka (Buchs, Switzerland), Imiquimodum (as Aldara™ cream) from 3M (Rüschlikon, Switzerland). Lipoteichoic acid (S.aureus and Streptococcus) were kindly provided by LUNAMeD AG (Zürich, Schwitzerland). Phosphorothioate modified CpG-ODN were synthesized by Microsynth (Balgach, Switzerland). Twelve days later, frequencies of p33-specific T cells was assessed in the blood by tetramer staining. The blood was collected into FACS buffer (PBS, 2% FBS, 5 mM EDTA) and lymphocytes were isolated by density gradient centrifugation for 20 min at 1200g and at 22°C in Lympholyte-M solution (Cedarlane Laboratories Ltd., Hornby, Canada). After washing the lymphocytes were resuspended in FACS buffer and stained for 10 min at 4°C with PE-labelled p33-H-2^{b} tetramer complexes and subsequently, for 30 min at 37°C, with anti-mouse CD8α-FITC antibody (Pharmingen, clone 53-6.7). Cells were analysed on a FACSCalibur using CellQuest software (BD Biosciences, Mountain View, CA).

Fig. 5 shows that various ligands for TLRs, with the exception of the TLR9 ligand CpGs, fail to enhance the T cell response against peptide p33 fused to the hepatis B core antigen (p33-VLPs). Mice were immunized with p33-VLPs in the presence of PBS or the indicated stimuli of TLRs. 100 ug HBc33 and 100 ug adjuvant were used. Frequencies of p33-specific T cells was assessed 8 days later by tetramer staining. Each bar representd one individual mouse. (LTA = Lipoteichonic acid, PGN = Peptidoglycan, LPS from E. coli K-235, Sigma).

### EXAMPLE 9

### Classical adjuvants such as Alum and IFA fail to enhance p33-specific CTL responses

Mice were immunized with 100 ug of p33-fused HBcAg (p33-VLPs) in the presence of CyCpGpt (20 nmol), Alum or IFA according to standard protocols) and 12 days later, mice were challenged with live LCMV (200 pfu) to assess anti-viral protection. Five days later, viral titers were assessed in the spleen.The spleens were ground with a homogenizer in Minimum Essential Medium (Gibco) containing 2 % fetal bovine serum and supplemented with glutamine, earls's salts and antibiotics (penicillin/streptomycin/amphotericin). The suspension was titrated in tenfold dilution steps onto MC57 cells. After incubation for one hour the cells were overlayed with DMEM containing 5% Fetal bovine serum, 1 % methyl cellulose, and antibiotics (penicillin /streptomycin /amphotericin). Following incubation for 2 days at 37°C the cells were assessed for LCMV infection by the intracellular staining procedure (which stains the viral nucleoprotein): Cells were fixed with 4 % Formaldehyde for 30 min followed by a 20 min lysing step with 1% Triton X-100. Incubation for 1 hour with 10 % fetal bovine serum blocked unspecific binding. Cells were stained with a rat anti-LCMV-antibody (VL-4) for 1 hour. A peroxidase-conjugated goat anti-rat-IgG (Jackson ImmunoResearch Laboratories, Inc) was used as secondary antibody followed by a colour reaction with ODP substrate according to standard procedures (Fig. 6).

Fig. 6 shows that the prototype adjuvants Alum and IFA fail to enhance VLP-induced immunity. Mice were vaccinested with p33-VLPs in the presence of PBS, CpGs, Alum or IFA and challenged 8 days later with live LCMV (200 pfu). Viral titers were determined 5 days later in the spleen.

### EXAMPLE 10

### Ligands for TLR4 enhance T cell response induced by VLPs loaded with CpGs.

Peptide p33 was coupled to Qb and loadad with CpG as in Example 7. The CpG used for this experiment was NK CpG (GGGGTCAACGTTGAGGGGG) (SEQ ID NO: 52). Mice were immunized subsequently with p33-Qb/CpG (180 ug) in the presence of PBS, MPL (Sigma, used according to the manufacturers instructions in a 1:1 mixture) or LPS (20 ug, E.coli K-235, Sigma). Ten days later, frequencies of p33-specific T cells was determined by tertramer staining (Fig 7A). The blood was collected into FACS buffer ( PBS, 2% FBS, 5 mM EDTA) and lymphocytes were isolated by density gradient centrifugation for 20 min at 1200g and at 22°C in Lympholyte-M solution (Cedarlane Laboratories Ltd., Hornby, Canada). After washing the lymphocytes were resuspended in FACS buffer and stained for 10 min at 4°C with PE-labelled p33-H-2^{b} tetramer complexes and subsequently, for 30 min at 37°C, with anti-mouse CDBα-FITC antibody (Pharmingen, clone 53-6.7). Cells were analysed on a FACSCalibur using CellQuest software (BD Biosciences, Mountain View, CA).

On the same day, mice were challenged ip with recombinant vaccina virus, expressing LCMV-GP (from which the peptide p33 is derived) and viral titers were assessed five days later in ovaries (Fig 7B). The ovaries were ground with a homogenizer in Minimum Essential Medium (Gibco) containing 5 % fetal bovine serum and supplemented with glutamine, earls's salts and antibiotics (penicillin/streptomycin/amphotericin). The suspension was titrated in tenfold dilution steps onto BSC40 cells. After overnight incubation at 37°C, the adherent cell layer was stained with a solution consisting of 50% Ethanol, 2% Crystal violet and 150mM NaCl for visualization of viral plaques.

Fig. 7 shows that ligands for TLR4 enhance CTL response against p33 coupled to VLPs loaded with CpGs. Mice were vaccinated with p33 coupled to Qb loaded with NK-PO CpGs in the presence of PBS, LPS or MPL (1:1 mixture). Eight days later, frequencies of p33-specific T cells were assessed by tetramer staining (A) On the same day, mice were challenged with recmombinant vaccina virus expressing LCMV-GP and viral titers were determined 5 days later in ovaries (B).

### EXAMPLE 11

### Conventional adjuvants fail to enhance T cell response induced by VLPs loaded with CpGs.

Peptide p33 is coupled to Qb and loaded with CpG as in Example 21. The CpG used for this experiment are NK CpG (GGGGTCAACGTTGAGGGGG (SEQ ID NO: 52) or G10-PO (GGGGGGGGGGGACGATCGTCGGGGGGGGGG) (SEQ ID NO: 54). Mice are immunized subsequently with p33-Qb/CpG (180 ug) in the presence of PBS, Alum or IFA, used according to standard protocols. Ten days later, frequencies of p33-specific T cells are determined by tertramer staining. The blood is collected into FACS buffer (PBS, 2% FBS, 5 mM EDTA) and lymphocytes are isolated by density gradient centrifugation for 20 min at 1200g and at 22°C in Lympholyte-M solution (Cedarlane Laboratories Ltd., Hornby, Canada). After washing the lymphocytes are resuspended in FACS buffer and stained for 10 min at 4°C with PE-labelled p33-H-2^{b} tetramer complexes and subsequently, for 30 min at 37°C, with anti-mouse CD8α-FITC antibody (Pharmingen, clone 53-6.7). Cells are analysed on a FACSCalibur using CellQuest software (BD Biosciences, Mountain View, CA).

On the same day, mice are challenged ip with recombinant vaccina virus, expressing LCMV-GP (from which the peptide p33 is derived) and viral titers are assessed five days later in ovaries. The ovaries are ground with a homogenizer in Minimum Essential Medium (Gibco) containing 5 % fetal bovine serum and supplemented with glutamine, earls's salts and antibiotics (penicillin/streptomycin/amphotericin). The suspension is titrated in tenfold dilution steps onto BSC40 cells. After overnight incubation at 37°C, the adherent cell layer is stained with a solution consisting of 50% Ethanol, 2% Crystal violet and 150mM NaCl for visualization of viral plaques..

**Table 1: Terminology and sequences of some of the immunostimulatory nucleic acids used throughout the specification.**

| Small letters indicate deoxynucleotides connected via phosphorothioate bonds while large letters indicate deoxynucleotides connected via phosphodiester bonds | | |
|---|---|---|
| Terminology | Sequence | SEQ ID NO |
| CyCpGpt | tccatgacgttcctgaataat | 11 |
| CpG-2006 | tcgtcgttttgtcgttttgtcgt | 48 |
| CyCpG | TCCATGACGTTCCTGAATAAT | 49 |
| B-CpGpt | tccatgacgttcctgacgtt | 50 |
| B-CpG | TCCATGACGTTCCTGACGTT | 51 |
| NKCpG | GGGGTCAACGTTGAGGGGG | 52 |
| CyCpG-rev-pt | attattcaggaacgtcatgga | 53 |
| g10gacga-PO (G10-PO) | GGGGGGGGGGGACGATCGTCGGGGGGGGGG | 54 |
| g10gacga-PS (G10-PS) | gggggggggggacgatcgtcgggggggggg | 55 |
| (CpG)200pA | | 56 |
| Cy(CpG)20 | | 57 |
| Cy(CpG)20-OpA | | 58 |

## Claims

1. A composition for enhancing an immune response in an animal comprising:
(a) a virus-like particle;
(b) an immunostimulatory DNA oligonucleotide which stimulates TLR9, wherein said DNA oligonucleotide is an unmethylated CpG-containing oligonucleotide packaged into said virus-like particle (a);
(c) at least one antigen, wherein said antigen is coupled to said virus-like particle (a); and
(d) at least one toll-like receptor (TLR) ligand activating a TLR 4, wherein said ligand is selected from the group consisting of: LPS and derivatives thereof, Monophosphoryl lipid A and derivatives thereof, and synthetic analoga of LPS.

2. The composition of claim 1, wherein the CpG motif of said unmethylated CpG-containing oligonucleotide is part of a palindromic sequence, wherein said palindromic sequence is GACGATCGTC (SEQ ID NO:39), and wherein said palindromic sequence is flanked at its 5'-terminus by at least 3 and at most 9 guanosine entities and wherein said palindromic sequence is flanked at its 3'-terminus by at least 6 and at most 9 guanosine entities, and wherein preferably said unmethylated CpG-containing oligonucleotide has a nucleic acid sequence selected from:
(a) GGGGACGATCGTCGGGGGG (SEQ ID NO:40);
(b) GGGGGACGATCGTCGGGGGG (SEQ ID NO:41);
(c) GGGGGGACGATCGTCGGGGGG (SEQ ID NO:42);
(d) GGGGGGGACGATCGTCGGGGGG (SEQ ID NO:43);
(e) GGGGGGGGACGATCGTCGGGGGGG (SEQ ID NO:44);
(f) GGGGGGGGGACGATCGTCGGGGGGGG (SEQ ID NO:45);
(g) GGGGGGGGGGACGATCGTCGGGGGGGGG (SEQ ID NO:46); and
(h) GGGGGGCGACGACGATCGTCGTCGGGGGGG (SEQ ID NO:47).

3. The composition of claim 1, wherein said unmethylated CpG-containing oligonucleotide consists of the sequence GGGGTCAACGTTGAGGGGG (SEQ ID NO:52).

4. The composition of claim 1, wherein said unmethylated CpG-containing oligonucleotide consists of the sequence GGGGGGGGGG GACGATCGTC GGGGGGGGGG (SEQ ID NO:54).

5. The composition of any one of the preceding claims, wherein said unmethylated CpG-containing oligonucleotide comprises 10 to 30 nucleotides.

6. The composition of any of the preceding claims, wherein said unmethylated CpG-containing oligonucleotide is a synthetic oligonucleotide.

7. The composition of any of the preceding claims, wherein said unmethylated CpG-containing oligonucleotide is not stabilized by phosphorothioate modifications of the phosphodiester backbone.

8. The composition of any one of the preceding claims, wherein said virus-like particle is a virus-like particle of RNA phage coat protein.

9. The composition of any one of claims 1 to 8, wherein said virus-like particle is a virus-like particle of bacteriophage Qβ coat protein.

10. The composition of claim 9, wherein said virus-like particle of Qβ coat protein is assembled exclusively from Qβ CP subunits (SEQ ID NO:1).

11. The composition of claim 8, wherein said RNA phage coat protein is the coat protein of bacteriophage AP205.

12. The composition of any one of the preceeding claims, wherein said antigen (c) is derived from the group consisting of:
(a) viruses;
(b) bacteria;
(c) parasites;
(d) prions;
(e) tumors;
(f) self-molecules;
(g) non-peptidic hapten molecules;
(h) allergens; and
(i) hormones.

13. The composition of any one of claims 1 to 11, wherein said antigen (c) is a tumor antigen, and wherein preferably said tumor antigen is selected from the group consisting of:
(a) Her2;
(b) GD2;
(c) EGF-R;
(d) CEA;
(e) CD52;
(f) human melanoma protein gp100;
(g) human melanoma protein melan-A/MART-1;
(h) tyrosinase;
(i) NA17-A nt protein;
(j) MAGE-3 protein;
(k) p53 protein;
(l) HPV16 E7 protein;
(m) an analogue of any of the antigens from (a) to (1); and
(n) antigenic fragments of any of the tumor antigens from (a) to (m).

14. The composition of any one of claims 1 to 11, wherein said antigen (c) is an allergen, and wherein preferably said allergen is derived from the group consisting of:
(a) pollen extract;
(b) dust extract;
(c) dust mite extract;
(d) fungal extract;
(e) mammalian epidermal extract;
(f) feather extract;
(g) insect extract;
(h) food extract;
(i) hair extract;
(j) saliva extract; and
(k) serum extract.

15. The composition of any one of the preceeding claims, for use in treatment of a disorder or disease comprising, and preferably selected from the group consisting of, allergies, tumors, chronic diseases and chronic viral diseases.

## Patentansprüche

1. Zusammensetzung zum Fördern einer Immunantwort in einem Tier, umfassend:
(a) ein virusartiges Partikel;
(b) ein immunstimulatorisches DNA-Oligonukleotid, welches TLR9 stimuliert, wobei das DNA-Oligonukleotid ein unmethyliertes CpG enthaltendes Oligonukleotid ist, verpackt in das virusartige Partikel (a);
(c) mindestens ein Antigen, wobei das Antigen an das virusartige Partikel (a) gekoppelt ist; und
(d) mindestens einen Toll-like Rezeptor- (TLR) -Ligand, der einen TLR 4 aktiviert, wobei der Ligand ausgewählt ist aus der Gruppe bestehend aus: LPS (Lipopolysacharid) und Derivaten davon, Monophosphoryl-Lipid A und Derivaten davon und synthetischen Analoga von LPS.

2. Zusammensetzung nach Anspruch 1, wobei das CpG-Motiv des unmethyliertes CpG enthaltenden Oligonukleotids Teil einer palindromischen Sequenz ist, wobei die palindromische Sequenz GACGATCGTC (SEQ ID NO: 39) ist, und wobei die palindromische Sequenz an ihrem 5'-Terminus durch mindestens 3 und höchstens 9 Guanosineinheiten flankiert wird, und wobei die palindromische Sequenz an ihrem 3'-Terminus durch mindestens 6 und höchstens 9 Guanosineinheiten flankiert wird, und wobei bevorzugt das unmethyliertes CpG enthaltende Oligonukleotid eine Nukleinsäuresequenz besitzt, ausgewählt aus:
(a) GGGGACGATCGTCGGGGGG (SEQ ID NO:40),
(b) GGGGGACGATCGTCGGGGGG (SEQ ID NO:41),
(c) GGGGGGACGATCGTCGGGGGG (SEQ ID NO:42);
(d) GGGGGGGACGATCGTCGGGGGG (SEQ ID NO:43);
(e) GGGGGGGGACGATCGTCGGGGGGG (SEQ ID NO:44);
(f) GGGGGGGGGACGATCGTCGGGGGGGG (SEQ ID NO:45);
(g) GGGGGGGGGGACGATCGTCGGGGGGGGG (SEQ ID NO:46); und
(h) GGGGGGCGACGACGATCGTCGTCGGGGGGG (SEQ ID NO: 47).

3. Zusammensetzung nach Anspruch 1, wobei das unmethyliertes CpG enthaltende Oligonukleotid aus der Sequenz GGGGTCAACGTTGAGGGGG (SEQ ID NO: 52) besteht.

4. Zusammensetzung nach Anspruch 1, wobei das unmethyliertes CpG enthaltende Oligonukleotid aus der Sequenz GGGGGGGGGG GACGATCGTC GGGGGGGGGG (SEQ ID NO: 54) besteht.

5. Zusammensetzung nach einem beliebigen der vorstehenden Ansprüche, wobei das unmethyliertes CpG enthaltende Oligonukleotid 10 bis 30 Nukleotide umfasst.

6. Zusammensetzung nach einem beliebigen der vorstehenden Ansprüche, wobei das unmethyliertes CpG enthaltende Oligonukleotid ein synthetisches Oligonukleotid ist.

7. Zusammensetzung nach einem beliebigen der vorstehenden Ansprüche, wobei das unmethyliertes CpG enthaltende Oligonukleotid nicht durch Phosphorthioatmodifikationen des Phosphodiesterrückgrats stabilisiert wird.

8. Zusammensetzung nach einem beliebigen der voranstehenden Ansprüche, wobei das virusartige Partikel ein virusartiges Partikel von RNA-Phagen-Hüllprotein ist.

9. Zusammensetzung nach einem beliebigen der Ansprüche 1 bis 8, wobei das virusartige Partikel ein virusartiges Partikel von Bakteriophage Qβ-Hüllprotein ist.

10. Zusammensetzung nach Anspruch 9, wobei das virusartige Partikel von Qβ-Hüllprotein ausschließlich aus Qβ-CP-Untereinheiten (SEQ ID NO: 1) zusammengebaut ist.

11. Zusammensetzung nach Anspruch 8, wobei das RNA-Phagen-Hüllprotein das Hüllprotein von Bakteriophage AP205 ist.

12. Zusammensetzung nach einem beliebigen der vorstehenden Ansprüche, wobei das Antigen (c) abgeleitet ist aus der Gruppe bestehend aus:
(a) Viren;
(b) Bakterien;
(c) Parasiten;
(d) Prionen;
(e) Tumoren;
(f) Selbst-Molekülen;
(g) nicht peptidischen Haptenmolekülen;
(h) Allergenen; und
(i) Hormonen.

13. Zusammensetzung nach einem beliebigen der Ansprüche 1 bis 11, wobei das Antigen (c) ein Tumorantigen ist, und wobei bevorzugt das Tumorantigen ausgewählt ist aus der Gruppe bestehend aus:
(a) Her2;
(b) GD2;
(c) EGF-R;
(d) CEA;
(e) CD52;
(f) humanem Melanomprotein gp100;
(g) humanem Melanomprotein melan-A/MART-1;
(h) Tyrosinase;
(i) NA17-A nt-Protein;
(j) MAGE-3-Protein;
(k) p53-Protein;
(l) HPV16 E7-Protein
(m) einem Analogon eines beliebigen der Antigene von (a) bis (1); und
(n) antigenen Fragmenten eines beliebigen der Tumorantigene von (a) bis (m).

14. Zusammensetzung nach einem beliebigen der Ansprüche 1 bis 11, wobei das Antigen (c) ein Allergen ist, und wobei bevorzugt das Allergen abgeleitet ist aus der Gruppe bestehend aus:
(a) Pollenextrakt;
(b) Staubextrakt;
(c) Staubmilbenextrakt;
(d) Pilzextrakt;
(e) epidermaler Extrakt von Säugern;
(f) Federextrakt;
(g) Insektenextrakt;
(h) Nahrungsmittelextrakt;
(i) Haarextrakt;
(j) Speichelextrakt; und
(k) Serumextrakt.

15. Zusammensetzung nach einem beliebigen der vorstehenden Ansprüche zur Verwendung in der Behandlung einer Störung oder Erkrankung umfassend, und bevorzugt ausgewählt aus der Gruppe bestehend aus Allergien, Tumoren, chronischen Erkrankungen und chronischen Viruserkrankungen.

## Revendications

1. Composition d'amélioration d'un réponse immunitaire chez un animal comprenant:
(a) une particule pseudo-virale;
(b) un oligonucléotide d'ADN immunostimulatoire qui stimule le TLR9, dans laquelle ledit oligonucléotide d'ADN est un oligonucléotide contenant des îlots CpG non méthylés intégré dans ladite particule pseudo-virale (a) ;
(c) au moins un antigène, dans laquelle ledit antigène est couplé à ladite particule pseudo-virale (a) ; et
(d) au moins un ligand d'un récepteur de type Toll (TLR) activant un TLR 4, dans laquelle ledit ligand est choisi dans le groupe consistant en: le LPS et des dérivés de celui-ci, le monophosphoryl Lipide A et des dérivés de celui-ci, et des analogues synthétiques du LPS.

2. Composition selon la revendication 1, dans laquelle le motif CpG dudit oligonucléotide contenant des îlots CpG non méthylés est une partie d'une séquence palindromique, dans laquelle ladite séquence palindromique est GACGATCGTC (SEQ ID NO: 39), et dans laquelle ladite séquence palindromique est flanquée à son extrémité 5' par au moins 3 et au plus 9 entités guanosine et dans laquelle ladite séquence palindromique est flanquée à son extrémité 3' par au moins 6 et au plus 9 entités guanosine, et dans laquelle de préférence ledit oligonucléotide contenant des îlots CpG non méthylés a une séquence d'acide nucléique choisie parme:
(a) GGGGACGATCGTCGGGGGG (SEQ ID NO: 40);
(b) GGGGGACGATCGTCGGGGGG (SEQ ID NO: 41);
(c) GGGGGGACGATCGTCGGGGGG (SEQ ID NO: 42);
(d) GGGGGGGACGATCGTCGGGGGG (SEQ ID NO: 43);
(e) GGGGGGGGACGATCGTCGGGGGGG (SEQ ID NO: 44);
(f) GGGGGGGGGACGATCGTCGGGGGGGG (SEQ ID NO: 45);
(g) GGGGGGGGGGACGATCGTCGGGGGGGGG (SEQ ID NO: 46); et
(h) GGGGGGCGACGACGATCGTCGTCGGGGGGG (SEQ ID NO: 47).

3. Composition selon la revendication 1, dans laquelle ledit oligonucléotide contenant des îlots CpG non méthylés consiste en la séquence GGGGTCAACGTTGAGGGGG (SEQ ID NO: 52).

4. Composition selon la revendication 1, dans laquelle ledit oligonucléotide contenant des îlots CpG non méthylés consiste en la séquence GGGGGGGGGG GACGATCGTC GGGGGGGGGG (SEQ ID NO: 54).

5. Composition selon l'une quelconque des revendications précédentes, dans laquelle ledit oligonucléotide contenant des îlots CpG non méthylés comprend 10 à 30 nucléotides.

6. Composition selon l'une quelconque des revendications précédentes, dans laquelle ledit oligonucléotide contenant des îlots CpG non méthylés est un oligonucléotide synthétique.

7. Composition selon l'une quelconque des revendications précédentes, dans laquelle ledit oligonucléotide contenant des îlots CpG non méthylés n'est pas stabilisé par des modifications phosphorothioate du squelette phosphodiester.

8. Composition selon l'une quelconque des revendications précédentes, dans laquelle ladite particule pseudo-virale est une particule pseudo-virale de protéine d'enveloppe de phage à ARN.

9. Composition selon l'une quelconque des revendications 1 à 8, dans laquelle ladite particule pseudo-virale est une particule pseudo-virale de protéine d'enveloppe de bactériophage Qβ.

10. Composition selon la revendication 9, dans laquelle ladite particule pseudo-virale de la protéine d'enveloppe de bactériophage Qβ est assemblée exclusivement à partir de sous-unités CP de Qβ (SEQ ID NO : 1).

11. Composition selon la revendication 8, dans laquelle ladite protéine d'enveloppe de phage à ARN est la protéine d'enveloppe du bactériophage AP205.

12. Composition selon l'une quelconque des revendications précédentes, dans laquelle ledit antigène (c) est dérivé du groupe consistant en :
(a) des virus ;
(b) des bactéries ;
(c) des parasites ;
(d) des prions ;
(e) des tumeurs ;
(f) des molécules du soi ;
(g) des molécules d'haptènes non peptidiques ;
(h) des allergènes ; et
(i) des hormones.

13. Composition selon l'une quelconque des revendications 1 à 11, dans laquelle ledit antigène (c) est un antigène tumoral, et dans laquelle préférentiellement ledit antigène tumoral est choisi dans le groupe consistant en :
(a) Her2 ;
(b) GD2 ;
(c) EGF-R ;
(d) CEA ;
(e) CD52 ;
(f) la protéine gp100 du mélanome humain ;
(g) la protéine melan-A/MART-1 du mélanome humain ;
(h) la tyrosinase ;
(i) la protéine NA17-A nt ;
(j) la protéine MAGE-3 ;
(k) la protéine p53 ;
(l) la protéine E7 de HPV 16 ;
(m) un analogue de l'un quelconque des antigènes de (a) à (1) ; et
(n) des fragments antigéniques de l'un quelconque des antigènes tumoraux de (a) à (m).

14. Composition selon l'une quelconque des revendications 1 à 11, dans laquelle ledit antigène (c) est un allergène, et dans laquelle préférentiellement ledit allergène est dérivé du groupe consistant en :
(a) un extrait de pollen ;
(b) un extrait de poussière ;
(c) un extrait d'acarien de poussière ;
(d) un extrait fongique ;
(e) un extrait d'épiderme de mammifère ;
(f) un extrait de plume ;
(g) un extrait d'insecte ;
(h) un extrait d'aliment ;
(i) un extrait de cheveu ;
(j) un extrait de salive ; et
(k) un extrait de sérum.

15. Composition selon l'une quelconque des revendications précédentes, pour une utilisation dans le traitement d'un trouble ou d'une maladie comprenant, et préférentiellement choisi(e) dans le groupe consistant en, des allergies, des tumeurs, des maladies chroniques et des maladies virales chroniques.
